(19) Europäisches Patentamt
European Patent Office
Office européen des brevets

(11)  EP 2 063 780 B1

(12)  EUROPEAN PATENT SPECIFICATION

(45) Date of publication and mention
of the grant of the patent:
**11.04.2018  Bulletin 2018/15**

(51) Int Cl.:
*A61B 5/117* (2016.01)

(21) Application number: **07837752.0**

(22) Date of filing: **06.09.2007**

(86) International application number:
**PCT/US2007/019378**

(87) International publication number:
**WO 2008/030481 (13.03.2008 Gazette 2008/11)**

(54) **IMAGING AND LOCATING SYSTEMS AND METHODS FOR A SWALLOWABLE SENSOR DEVICE**

ABBILDUNGS- UND LOKALISIERUNGSSYSTEME UND -VERFAHREN FÜR EINE SCHLUCKBARE SENSORENVORRICHTUNG

SYSTÈMES ET PROCÉDÉS D'IMAGERIE ET DE LOCALISATION POUR DISPOSITIF DE CAPTEUR AVALABLE

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **06.09.2006  US 842360 P**
**05.06.2007  US 924928 P**

(43) Date of publication of application:
**03.06.2009  Bulletin 2009/23**

(73) Proprietor: **Innurvation, Inc.**
**Columbia, MD 21046-1169 (US)**

(72) Inventors:
• **BANDY, William, R.**
**Gambrills, MD 21054 (US)**
• **DAVENPORT, Roger, A.**
**Plantation, FL33322 (US)**
• **POWELL, Kevin, J.**
**Annapolis, MD 21043 (US)**
• **ARNESON, Michael, R.**
**Finksburg, MD 21048 (US)**

(74) Representative: **Jehle, Volker Armin et al**
**Bosch Jehle Patentanwaltsgesellschaft mbH**
**Flüggenstrasse 13**
**80639 München (DE)**

(56) References cited:
**WO-A2-2006/045011     US-A1- 2004 068 204**
**US-B2- 6 904 308**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

BACKGROUND OF THE INVENTION

Field of the Invention

The present invention generally relates to medical diagnostics and/or treatment. More particularly, the present invention generally relates to swallowable medical diagnostic and/or treatment devices and methods. The present invention is set out in the appended claims.

Background Art

A swallowable sensor device is a medical diagnostic device that may be ingested by a patient. For example, a swallowable sensor device can be used to collect data regarding a patient's internal body chemistry. This data can then be transmitted to an external device for diagnostic purposes. Such a diagnostic technique is easy to administer and less invasive compared to conventional diagnostic techniques, such as surgery.

Despite the potential benefits, conventional swallowable sensor devices have several drawbacks. One drawback is that conventional swallowable sensor devices use a radio frequency (RF) signal platform to collect data and transmit the data to external entities. The RF signal platform is problematic for several reasons.

First, the extent to which RF signals cause harm to human tissue is not fully understood. The potential for harm only increases if the source of the RF signals comes closer to the human tissue. As a result, many patients are apprehensive about ingesting a device that emits RF signals.

Second, swallowable sensor devices based on an RF signal platform are quite large because a relatively high powered RF signal is required to overcome the relatively short attenuation length of RF signals in the body. In fact, conventional swallowable sensor devices are so large that a portion of the patient population cannot even swallow these devices; and if it can be swallowed, the large size of a conventional swallowable sensor device may cause it to become lodged in a patient's gastrointestinal tract, which would require surgery to remove.

Third, because the RF signal travels at the speed of light, the time difference of arrival at closely spaced receivers is too small to use to determine the location of the RF signal source.

Thus, what is needed are improved diagnostic and treatment devices and methods. US Patent N°. 2004/0068204 describes a device and method for mapping, diagnosing and treating the intestinal tract using a capsule passing through the intestinal tract; further, a capsule tracking system is provided for tracking a capsule's location along the length of the intestinal tract.

BRIEF DESCRIPTION OF THE DRAWINGS/FIGURES

The accompanying drawings, which are incorporated herein and form part of the specification, illustrate the present invention and, together with the description, further serve to explain the principles of the invention and to enable a person skilled in the relevant art(s) to make and use the invention.

FIG. 1 illustrates a swallowable sensor device disposed in a human according to an embodiment.
FIG. 2 is a block diagram of a swallowable sensor device according to an embodiment.
FIG. 3 is a block diagram of a communications module according to an embodiment.
FIG. 4 is a block diagram of a swallowable sensor device according to another embodiment.
FIG. 5 is a block diagram of a communications network according to an embodiment.
FIG. 6 is a block diagram of an exemplary communications network utilizing a sensor link module according to an embodiment.
FIG. 7 is a block diagram of a sensor link module according to an embodiment.
FIG. 8 illustrates sensing elements included in a sensor link module according to an embodiment.
FIG. 9 illustrates a counter and transducer of a sensing element according to an embodiment.
FIG. 10 illustrates a piezoelectric element of a transducer according to an embodiment.
FIG. 11 illustrates an exemplary computer system useful for implementing an embodiment.
FIG. 12 illustrates a plurality of sensor link modules positioned on a patient in accordance with an embodiment.
FIG. 13 depicts a block diagram illustrating an example method for locating a swallowable sensor device according to embodiments.
FIG. 14 illustrates example geometry useful for determining the location of a swallowable sensor device according to an embodiment.
FIG. 15 illustrates details of the example geometry depicted in FIG. 14.

FIGS. 16 and 17 illustrate example geometry useful for determining the location of a swallowable sensor device according to embodiments.

FIGS. 18 depicts a block diagram illustrating an example method for internally imaging a patient according to embodiments.

FIGS. 19A and 19B illustrate example geometry useful for imaging an object based on a first and second acoustic signal transmitted from a swallowable sensor device according to embodiments.

FIGS. 20A and 20B illustrate example geometry useful for imaging an object based on a first and second acoustic signal transmitted from an external device according to embodiments.

FIGS. 21A and 21B illustrate example geometry useful for imaging an object based on a first and second acoustic signal transmitted from an external device according to other embodiments.

FIGS. 22A, 22B and 22C illustrate example geometry useful for imaging an object based on a first and second acoustic signal transmitted from a swallowable sensor device according to other embodiments.

FIG. 23 illustrates example geometry useful for computing coordinates of a voxel according to an embodiment.

The features and advantages of the present invention will become more apparent from the detailed description set forth below when taken in conjunction with the drawings, in which like reference characters identify corresponding elements throughout. In the drawings, like reference numbers generally indicate identical, functionally similar, and/or structurally similar elements. The drawing in which an element first appears is indicated by the leftmost digit(s) in the corresponding reference number.

DETAILED DESCRIPTION OF THE INVENTION

I. Introduction
II. Overview

A. An Example Environment
B. An Example Swallowable Sensor Device
C. Example External Entities Coupled To A Swallowable Sensor Device
D. Example Computer System Embodiments

III. Locating A Swallowable Sensor Device Disposed Within A Patient In Accordance With An Embodiment

A. Positioning Of Sensor Link Modules On A Patient In Accordance With An Embodiment
B. An Example Locating Method
C. Example Calculations To Determine The Location Of A Swallowable Sensor Device Using Phased Array Receivers
D. Example Calculations To Determine The Location Of A Swallowable Sensor Device Using Single Element Receivers

IV. Internal Imaging In Accordance With An Embodiment

A. Example Methods For Internally Imaging A Patient
B. Image Capture For Three Dimensional Viewing
C. Image Creation

V. Conclusion

I. Introduction

The present invention is directed to locating and imaging with a swallowable sensors. In the specification, reference to "one embodiment," "an embodiment," "an example embodiment," etc., indicate that the embodiment described may include a particular feature, structure, or characteristic, but every embodiment may not necessarily include the particular feature, structure, or characteristic. Moreover, such phrases are not necessarily referring to the same embodiment. Further, when a particular feature, structure, or characteristic is described in connection with an embodiment, it is submitted that it is within the knowledge of one skilled in the art to affect such feature, structure, or characteristic in connection with other embodiments whether or not explicitly described.

An embodiment is directed to locating a swallowable sensor device as it travels through a patient. A system in accordance with this embodiment includes a swallowable sensor device, a plurality of sensing elements, and a computation module. The swallowable sensor device transmits an acoustic signal. The plurality of sensing elements receive the acoustic signal at respective times. The computation module computes a location of the swallowable sensor device based on the respective times and a reference time. The reference time is established when a first sensing element receives the acoustic signal, wherein the first sensing element receives the acoustic signal before at least a subset of the other sensing elements. For example, the first sensing element can be the sensing element closest to the swallowable sensor device, whereby the first sensing element would be the first to receive the acoustic signal.

Another embodiment is directed to imaging an interior portion of a patient. An example system in accordance with this embodiment includes a swallowable sensor device and a plurality of sensing elements. The swallowable sensor device transmits a first acoustic signal from a first location and a second acoustic signal from a second location. The plurality of sensing elements receive the first and second acoustic signals. An image is formed from the first and second acoustic signals in accordance with one of two examples. In a first example, the plurality of sensing elements include detectors that capture two two-dimensional images of an interior portion of the patient based on the received acoustic signals. The two two-dimensional images are stereoscopically displayed to form a three-dimensional image. In a second example, computation logic computes a three-dimensional volume element of an interior portion of the patient based on the received acoustic signals.

Each of these embodiments is described in more detail below. Before describing these embodiments, however, an overview of the swallowable sensor device is provided.

II. Overview

To better understand the locating and imaging methods, systems, and apparatuses, it is helpful to describe (A) an example environment in which such methods, systems, and apparatuses may be implemented, (B) an example swallowable sensor device, (C) example external devices that may be coupled to such a swallowable sensor device, and (D) example computer system embodiments, as set forth below.

A. An Example Environment

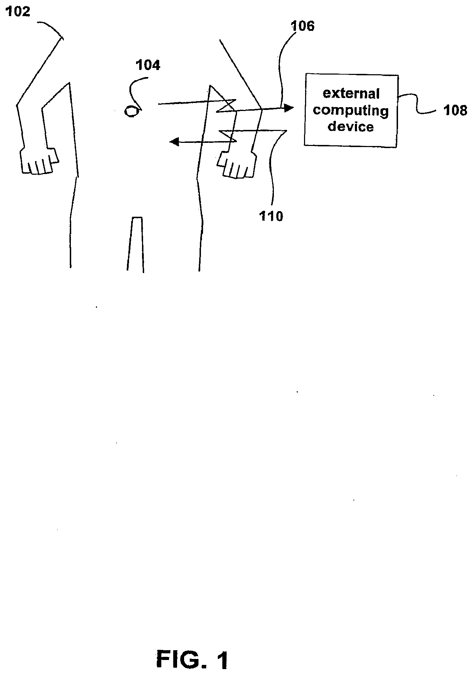

FIG. 1 shows a swallowable sensor device 104 disposed in a patient 102 according to an embodiment. Swallowable sensor device 104 is configured to sense one or more attributes or conditions of patient 102 as swallowable sensor device 104 passes through patient 102. While passing through patient 102, swallowable sensor device 104 transmits an acoustic signal 106 to be received outside patient 102. As shown in FIG. 1, an external computing device 108 may receive acoustic signal 106. Based on the received acoustic signal, computing device 108 may determine the location of swallowable sensor device 104 and image an interior portion of patient 102. Computing device 108 may also decode information encoded in acoustic signal 106, to interact with the information, to process the information, and/or to transmit the information (raw or processed) to another entity.

In an embodiment, computing device 108 can interact with swallowable sensor device 104. Such interaction may be used to control functions of swallowable sensor device 104 and/or to image an internal portion of a patient, as described in more detail below. As shown in FIG. 1, computing device 108 may interact with swallowable sensor device 104 by, for example, transmitting a communication signal 110 to be received by swallowable sensor device 104.

In embodiments, patient 102 may be provided with one or more swallowable sensor devices 104 that patient 102 may at designated times and/or periodically swallow to perform an analysis of one or more health-related conditions of patient 102.

B. An Example Swallowable Sensor Device

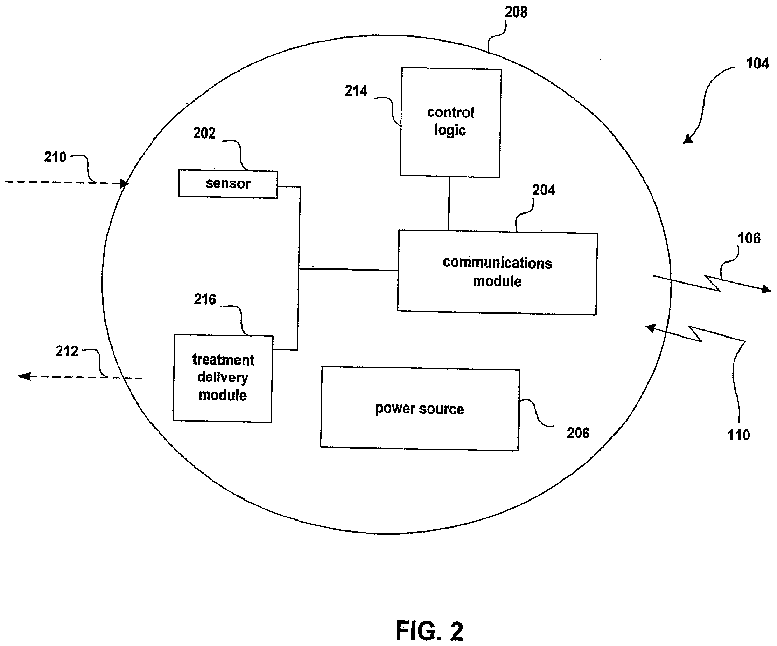

FIG. 2 shows an example block diagram of swallowable sensor device 104, according to an embodiment. In FIG. 2, swallowable sensor device 104 includes a housing 208 that holds one or more sensors 202, a communications module 204, control logic 214, and a power source 206. Each of these elements is described in more detail below.

Housing 208 contains sensor(s) 202, communications module 204, and power source 206, and is configured to be swallowable by or inserted within a human and/or animal. Housing 208 may be the size of a vitamin or other type of pill that is swallowable by humans. Housing 208 may be any suitable shape, including oval, elliptical (as shown in FIG. 2), capsule shaped, or spherical. The small size of housing 208 allows swallowable sensor device 104 to be easily ingested by an average patient 102. The small size overcomes difficulties present with existing pills that emit RF radiation (such as camera pills), which are often so large that they present a difficulty in swallowing. Further, the small size of housing 208 allows swallowable sensor device 104 to pass completely through the digestive system of a patient 102 without becoming trapped due to size incompatibility.

Housing 208 may be made from a variety of non-digestible or slow rate of digestion materials, including: a plastic material, such as a resin, a resinoid, a polymer, a cellulose derivative, a casein material, and/or a protein; a metal, including a combination of metals/alloy; a glass material; a ceramic; a composite material; and/or other material/combination of materials. In a particular embodiment, housing 208 may be comprised of a material that aids in the sensing of biological, chemical, or other attributes of body material that touches or comes in close proximity to the housing 208, such as could be called an integrated housing and sensor material.

Swallowable sensor device 104 also includes a sensors 202 and a treatment delivery module 216. Although FIG. 2 illustrates swallowable sensor device 104 as having a single sensor 202 and treatment delivery module 216, one of skill in the art will recognize that other numbers of sensors and treatment delivery modules may be included in swallowable sensor device 104.

Sensor 202 is used to sense (*e.g.*, measure, detect, etc.) a received stimulus 210, and generate a sensor output signal. The sensor output signal may be a digital or analog signal, depending on the particular implementation of sensor 202, that is received by communications module 204. In alternative embodiments the housing 208 may be made of sensor 202, or sensor 202 may be integrated within the materials known as housing 208. Sensor 202 may be configured to sense received stimulus 210 based on the location of swallowable sensor device 104.

Treatment delivery module 216 is used to deliver (*e.g.*, administer, emit, etc.) a treatment 212. Treatment delivery module 216 may be configured to deliver treatment 212 based on the location of swallowable sensor device 104.

Communications module 204 receives the sensor output signal, and generates acoustic signal 106 to include data based on sensor output signal 212. Acoustic signal 106 is transmitted from swallowable sensor device 104. Communications module 204 may also receive communication signal 110 transmitted from an external device, such as external computing device 108. Received communication signal 110 may instruct sensor 202 to receive stimulus 210 from the surrounding environment based on the location of swallowable sensor device 104, and may instruct treatment delivery module 216 to deliver treatment 212 to the surrounding environment based on the location of swallowable sensor device 104.

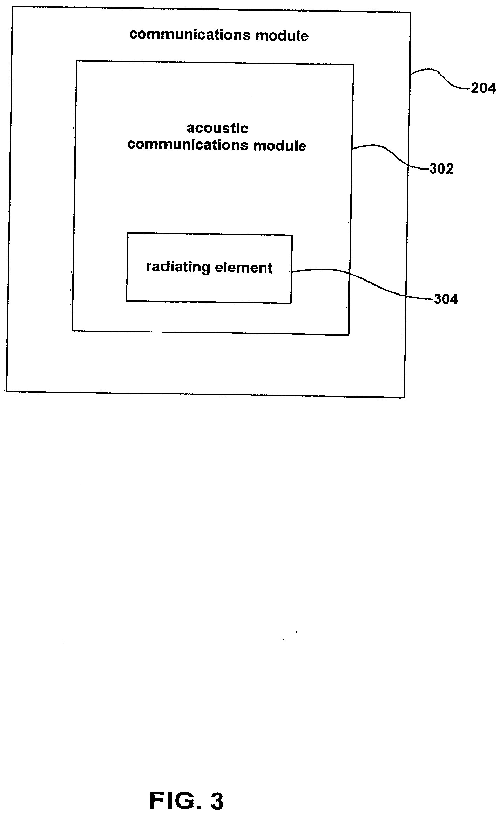

FIG. 3 depicts an example embodiment of an acoustic communications module 302 included in swallowable sensor device 104. Acoustic communication module 302 is configured to transmit and/or receive an acoustic communications signal. For example, acoustic communications module 302 may include an acoustic transmitter and/or acoustic receiver. In this example, sensor output signal 212 is modulated on an acoustic signal that is transmitted as communications signal 106 by the acoustic transmitter. The acoustic communications signal 106 may be transmitted by radiating element 304. In a similar manner, communication signal 110 may be received by the acoustic receiver (not shown).

The acoustic transmitter and/or acoustic receiver may be, for example, a piezoelectric (PZT) element or transducer that vibrates at acoustic frequencies. An example acoustic frequency range in which acoustic communication signals 106 and 110 may be transmitted is 20 Hz to 16 KHz, although the frequency may be an acoustic frequency higher or lower than this range in some applications. In a likewise fashion, acoustic communications module 302 may include an ultrasonic communications module, configured to transmit and/or receive a communications signal at ultrasonic frequencies (e.g., greater than 20KHz).

Communications module 204 may be configured to modulate information from sensor 202 or other information according to a variety of modulation techniques, including amplitude modulation (AM), frequency modulation (FM), and phase modulation (PM), and including any combination of these modulation techniques, including quadrature modulation schemes, or any other modulation techniques.

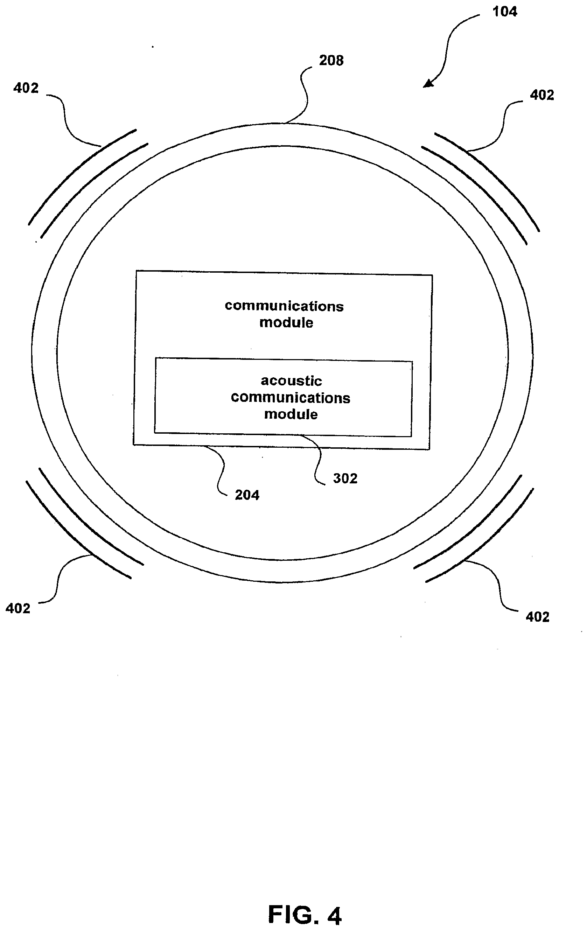

FIG. 4 shows a view of swallowable sensor device 104, with communications module 204 including acoustic communications module 302. In FIG. 4, communications module 204 is coupled to housing 208. Housing 208 vibrates according to acoustic communications module 302 to transmit a communications signal 402, which is an acoustic version of communications signal 106. In FIG. 4, housing 208 functions as an acoustic radiating element, vibrating at acoustic frequencies according to acoustic communications module 302.

Returning to FIG. 2, swallowable sensor device 104 also includes control logic 214, which may be used to gate or control swallowable sensor device 104. Control logic 214 may operate in a sub-threshold voltage (Vt) manner (e.g., to save power), or may operate in normal bias modes. In an embodiment, swallowable sensor device 104 is an autonomous device with one way communication (transmission capability), so that control logic 214 may be extremely simple, and thus would not consume much power even when operating in normal bias modes. However, in another embodiment, swallowable sensor device 104 may communicate in both directions-i.e., it may be configured to transmit information to and receive instructions from computing device 108. Control logic 214 may thus have additional complexity in order to, for example, decode and implement received instructions. In a further embodiment, control logic 214 may a computation module (not shown) that is configured to determine a location of swallowable sensor device 104 and/or to image an internal portion of patient 102, as described in more detail below.

Swallowable sensor device 104 also includes power source 206. Power source 206 provides power (e.g., via electrical energy) to operate the components of swallowable sensor device 104 that require power, such as communications module 204 and/or sensor 202. Power source 206 may include, for example and without limitation, a battery, a liquid or gel surrounding communications module 204, or an energy harvesting module.

In an embodiment, swallowable sensor device 104 is configured for low power operation, including extreme low power (XLP) operation. To achieve XLP operation, swallowable sensor device 104 can use one or both of a very small battery and energy harvesting to operate swallowable sensor device 104. In an embodiment, circuits of swallowable sensor device 104 are implemented in one or more integrated circuits (ICs), in a technology such as CMOS, or other technology. The IC(s) and any other internal components of swallowable sensor device 104 may be mounted to a circuit board, or mounted directly to housing 208. Thus, in embodiments, power source 206 is configured for low power output, including supplying power in the milliwatt and microwatt ranges. Such low power requirements enable the size of power source 206 to be minimal.

In a CMOS embodiment, MOSFET circuits may be configured to operate in a deep sub-threshold voltage (sub-Vt) mode, which lowers their switching time to acoustic switching frequencies, and lowers their power consumption by orders of magnitude. In such a mode the MOSFET devices operate as analog devices. Such operation was demonstrated in the mid-1980's by Carver Meade with regard to eye and ear chips. Such a mode of operation eliminates the need for digitizing the sensor data, which can be very power intensive, and which further reduces the power consumption by a large factor.

After being swallowed by patient 102, swallowable sensor device 104 eventually passes from patient 102, such as when patient 102 has a bowel movement to excrete waste. In an embodiment, swallowable sensor device 104 is disposable. In another embodiment, swallowable sensor device 104 may be recovered, (and recycled) for reuse.

Depending upon the ability or control of the patient, swallowable sensor device 104 may alternatively be inserted into a lower gastrointestinal tract of patient 102 as a suppository device.

Depending on the configuration of sensor 202, while passing through patient 102, swallowable sensor device 104 can sense conditions and/or features of any part of the gastrointestinal tract, and any of the materials/fluids contained within and/or secreted by the organs in the gastrointestinal tract or organs indirectly associated with the gastrointestinal tract. Swallowable sensor device 104 can also receive conditions or signals from even more remote body organs such as acoustic pickup of heartbeat and/or breathing and more indirect conditions such as temperature. In an embodiment, a camera or other imaging device is coupled to swallowable sensor device 104 to allow visual observation of patient 102.

C. Example External Entities Coupled To A Swallowable Sensor Device

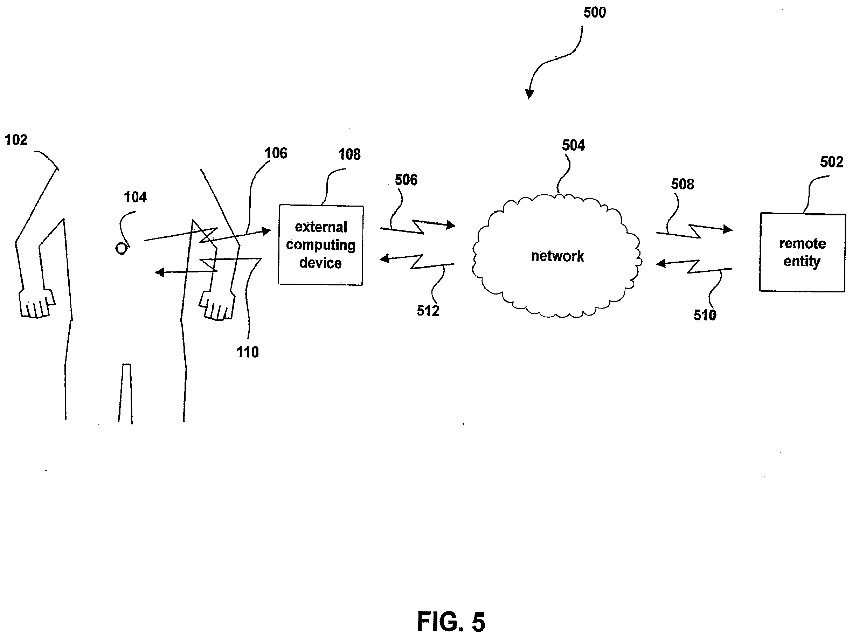

As mentioned, swallowable sensor device 104 transmits information in acoustic signal 106 to be received outside patient 102, such as by computing device 108. In an embodiment, computing device 108 may be configured to communicate with a remote entity 502, such as shown in an example sensor communications network 500 of FIG. 5. Computing device 108 may be configured to communicate with remote entity 502 using wired and/or wireless links, in a direct fashion or through a network 504. For example, computing device 108 transmits a communication signal 506 to network 504, which transmits a communication signal 508 to remote entity 502. Network 504 may be any type of network or combination of networks, such as a telephone network (e.g., a land line and/or cellular network), a personal area network (PAN), a local area network (LAN), and/or a wide area network (WAN) such as the Internet.

Remote entity 502 may be one or more of a variety of entities, including a human and/or computer-based entity. For example, remote entity 502 may include a doctor who receives information collected by swallowable sensor device 104 (and optionally processed by computer device 108) in communication signal 508.

As shown in FIG. 5, sensor communications network 500 may include a return communications path from remote entity 502 through network 504 to computing device 108. For example, a return communication signal 510 is transmitted by remote entity 502 to network 504, which transmits a return communication signal 512 to computing device 108. In this manner, remote entity 502 (e.g., doctor and/or computer system) can provide feedback to computing device 108 in communication signal 512 regarding the analysis of patient 102 performed by swallowable sensor device 104. Return communication signal 512 may include any type of data/information format for providing the feedback, including an email, a text message, a text file, a document formatted for commercially available word processing software, a proprietary document/data format, auditory alarms, alerts and messages, etc. In addition, computing device 108 may send instructions to swallowable sensor device 104 in communication signal 110 based on the feedback provided from remote entity 502 via network 504.

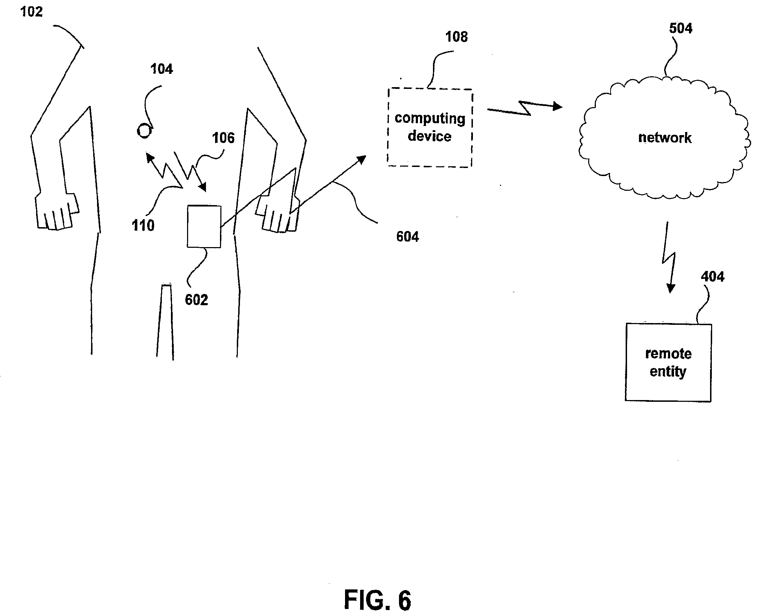

Swallowable sensor device 104 may communicate with computing device 108 via an intermediate sensor link module 602, as shown in FIG. 6. Sensor link module 602 receives acoustic signal 106 from swallowable sensor device 104. As shown in FIG. 6, sensor link module 602 is coupled to patient 102. In an embodiment, sensor link module 602 includes one or more modules that determine the location of swallowable sensor device 104 and/or image an interior portion of patient 102 based on acoustic signal 106 received from swallowable sensor device 104.

In another embodiment, sensor link module 602 transmits a communication signal 604 to computing device 108, to provide the information from swallowable sensor device 104 to computing device 108. In this embodiment, computing device 108 includes one or more modules that determine the location of swallowable sensor device 104 and/or image an interior portion of patient 102 based on acoustic signal 106 received from swallowable sensor device 104.

In a further embodiment, sensor link module 602 may provide a communication interface between swallowable sensor device 104 and network 504, such that a separate computing device 108 is not required. In such an embodiment, sensor link module 602 may perform functions of computing device 108 described above, and thus sensor link module 602 may be referred to as a computing device. For example sensor link module 602 may receive acoustic signal 106 from and transmit communication signal 110 to swallowable sensor device 104.

Multiple sensor link modules 602 are used to determine the location of swallowable sensor device 104 and to image an interior portion of patient 102, as described in more detail below. In an embodiment, multiple sensor link modules 602 may be attached to patient 102 at various locations in order to receive the interior acoustic signal from different angles. Sensor link module 602 may be, for example, directly attached to the skin of patient 102, such as by an adhesive or a strap. Alternatively, multiple sensor link modules 602 may be embedded in a wearable fabric that is worn by patient 102. Sensor link module 602 may be attached to patient 102 in one or more locations, including the head, neck, chest, back, abdomen, arm, leg, etc. With regard to receiving acoustic signal 106 from swallowable sensor device 104 passing through the gastrointestinal tract, sensor link module 602 may be attached to the neck, chest, back, and/or abdomen for a short signal path. In an embodiment, a plurality of sensor link modules are coupled to a front portion of patient 102 to reduce distortion caused by bones in the back portion of patient 102.

An amount of received information is proportional to the number of sensor link modules 602 attached to patient 102. The array of sensor link modules 602 may be attached at specific locations on patient 102 to increase, and even maximize, the received diagnostic information. Multiple sensor link modules 602 can identify a specific location of the swallowable sensor device which can be used for linking a location to the detection of a sensed material. The location can also be used to identify a historical analysis of the track taken by the swallowable device and the speed of passage.

For example, the attachment of an array of three or more sensor link modules 602 to patient 102 may enable triangulation or other location finding algorithms to be used to locate swallowable sensor device 104 in patient 102. Alternatively, one or more sensor link modules 602 having three or more sensing elements that may be used to the same effect. By locating swallowable sensor device 104 in patient 102, a location of a sensed material in patient 102 can be determined.

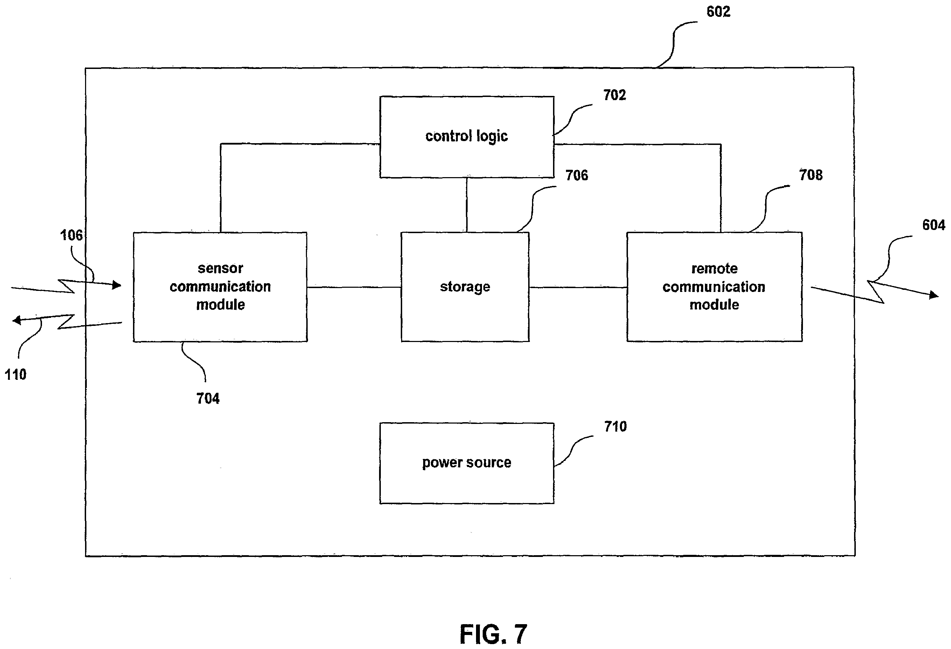

In embodiments, sensor link module 602 may be configured in various ways. For instance, FIG. 7 shows an example sensor link module 602, according to an embodiment. As shown in FIG. 7, sensor link module 602 includes a sensor communication module 704, storage 706, control logic 702, a remote communication module 708, and a power source 710.

Sensor communication module 704 receives acoustic signal 106 from and transmits communication signal 110 to swallowable sensor device 104. Sensor communication module 704 demodulates the sensor-related data of acoustic signal 106. Furthermore, sensor communication module 704 may process and/or convert a format of the data received in acoustic signal 106. For example, sensor communication module 704 may perform an analog-to-digital (A/D) conversion of the received sensor data, and output a sensor data signal. The sensor data signal may be received by storage 706 and/or by control logic 702.

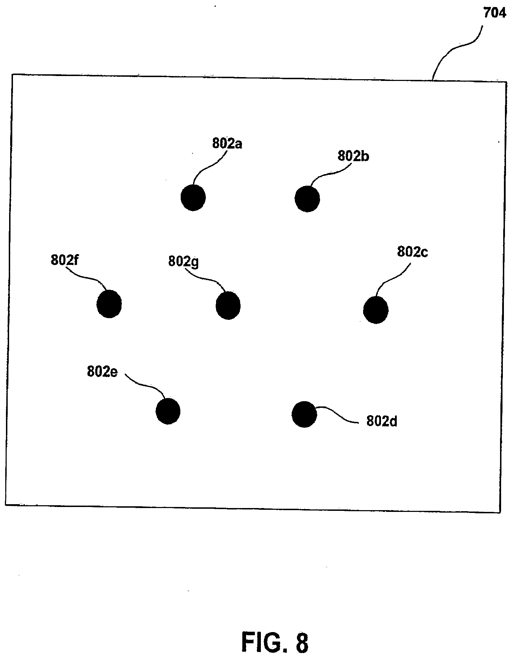

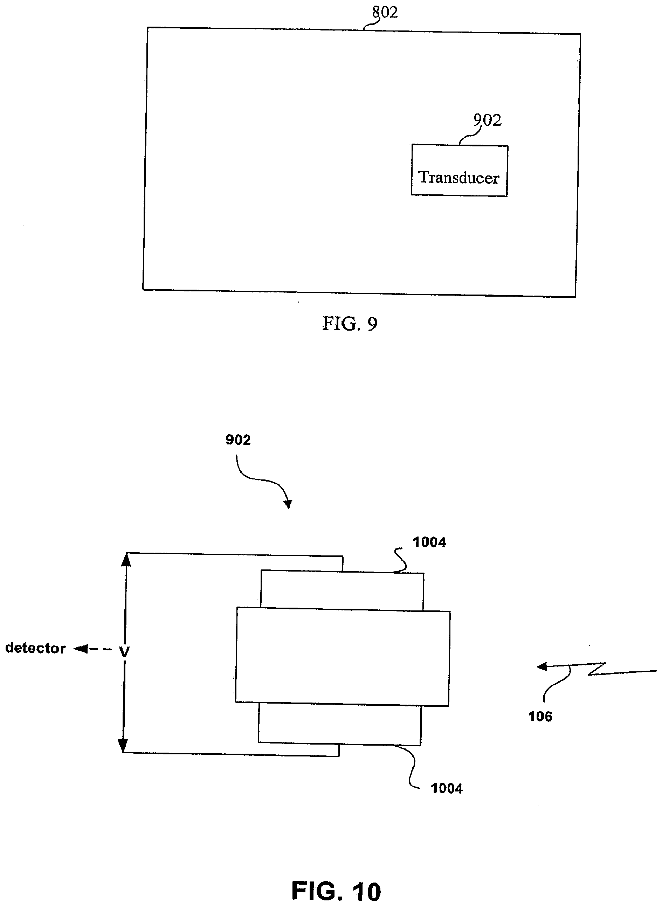

Referring to FIG. 8, sensor communication module 704 may include a plurality of sensing elements 802a-g that are configured to respond to acoustic signal 106. Sensing elements 802 may be configured in a plurality of orientations, including, for example, a hexagonal close pack configuration, as illustrated in FIG. 8. In an embodiment, each sensing element 802 includes a transducer 902, as illustrated in FIG. 9.

Transducer 902 is a device that receives a signal in one form of energy and converts it into a signal in another form of energy. In an embodiment, transducer 902 can convert mechanical energy into electrical energy and vice versa. For example, transducer 902 may receive acoustic signal 106 and convert it into an electrical signal. In such an example, transducer 902 may comprise an element 1004 that responds to acoustic signal 106 to generate a voltage, V. The voltage is detectable as an electric signal by a detector (*e.g.*, charge coupled device (CCD) or direct conversion receiver), as illustrated in FIG. 10. Because transducer 902 can convert acoustic signal 106 into an electric signal, each sensing element may serve as a pixel for generating a two dimensional image of an interior portion of patient 102, as described in more detail below.

Element 1004 may comprise, for example, a ceramic (such as lead zirconium titanate (PZT) or barium titanium (BaTi)), a piezo-polymer (such as polyvinylidene fluoride (PVDF)), a single crystalline (such as lithium nitrite (LiN), lithium titanate (LiTi), a film (such as zinc oxide (ZnO)), or some other type of material for converting mechanical energy into electrical energy and vice versa.

Storage 706 is configured to store data received by swallowable sensor device 104. Storage 706 may include any type of suitable storage, including a hard drive and/or memory devices. Storage 706 can output the stored data in a stored sensor data signal, for subsequent transmission to computing device 108 by remote communication module 708.

Control logic 702 is configured to control operation of sensor link module 602. Furthermore, control logic 702 may be configured to perform computations to determine the location of swallowable sensor device and/or to image an internal portion of patient 102, as described in more detail below. Additionally, control logic 702 may include a counter to determine when acoustic signal 106 is received from swallowable sensor device 104.

Remote communication module 708 transmits the data, which is stored in storage 706, in communication signal 604. Remote communication module 708 may be configured to transmit communication signal 604 in a variety of formats/protocols, such as a standard RF communication protocol including Bluetooth, IEEE 802.11, Zigbee, or other communication protocol, standard or otherwise. For example, in embodiments, computing device 108 may be a Bluetooth, 802.11, and/or Zigbee configured handheld device such as cell phone, personal digital assistant (PDA), a Blackberry™, wrist watch, music player, or laptop, or other type of computer, handheld, desktop, or other device.

Power source 710 provides power to elements of sensor link module 602 that require power, such as control logic 702, sensor communication module 704, storage 706, and remote communication module 708. For example, power source 710 may include one or more batteries that are rechargeable or non-rechargeable. Power source 710 may also (or alternatively) include an interface for externally supplied power, such as standard A/C power. Power source 710 may also (alternatively) comprise solar cells or a hand powered generator.

As described above, in an embodiment, swallowable sensor device 104 can transmit an acoustic signal. By receiving the acoustic signal transmitted by swallowable sensor device 104, sensor link module 602 may perform a type of ultrasound analysis based on the human interior generated acoustic signal from swallowable sensor device 104. As acoustic signal 106 is transmitted through patient 102 from swallowable sensor device 104, signal 106 is transformed by attenuation, refraction, and reflection, as a function of the tissue of patient 102 that signal 106 passes through. The transformed signal thus provides additional diagnostic information to sensor link module 602, very much like a diagnostic ultrasound conveys diagnostic information that can be analyzed by a trained technician. The acoustic signal from swallowable sensor device 104 may be viewed as an "interior" ultrasound or "sonogram", which can be analyzed to extract additional diagnostic information regarding patient 102. In an embodiment, information received by sensor link module 602 regarding the interior ultrasound signal can be used to generate a graphical display of at least a portion of the interior of patient 102, as described in more detail below.

D. Example Computer System Embodiments

According to an example embodiment, swallowable sensor device 104 may execute computer-readable instructions to perform its functions. Furthermore, sensor link module 602 may execute computer-readable instructions to communicate with swallowable sensor device 104. For example, sensor link module 602 may execute computer-readable instructions to determine the location of swallowable sensor device 104 and image an interior portion of patient 102. Still further, a computing device may execute computer-readable instructions to communicate with swallowable sensor device 104 and/or sensor link module 602, and/or to process data obtained by swallowable sensor device 104 and/or sensor link module 602, as described above. Still further, a test kit and medical diagnostic network system may each execute computer-readable instructions to perform its functions.

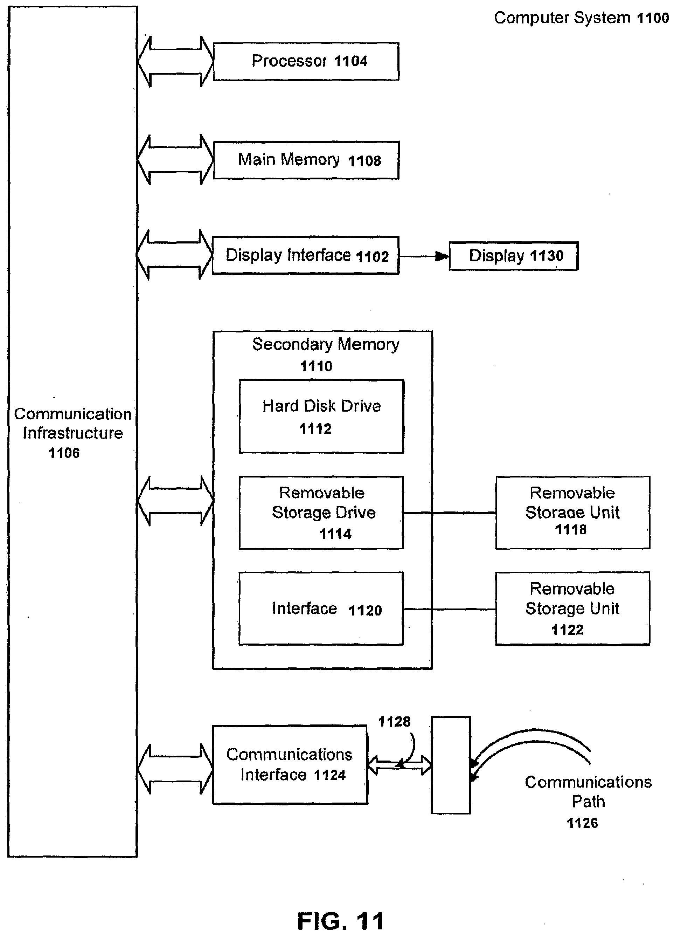

In one embodiment, one or more computer systems are capable of carrying out the functionality described herein. An example computer system 1100 is shown in FIG. 11.

The computer system 1100 includes one or more processors, such as processor 1104. The processor 1104 is connected to a communication infrastructure 1106 (e.g., a communications bus, cross-over bar, or network). Various software embodiments are described in terms of this exemplary computer system. After reading this description, it will become apparent to a person skilled in the relevant art(s) how to implement the invention using other computer systems and/or architectures.

Computer system 1100 can include a display interface 1102 that forwards graphics, text, and other data from the communication infrastructure 1106 (or from a frame buffer not shown) for display on the display unit 1130.

Computer system 1100 also includes a main memory 1108, preferably random access memory (RAM), and may also include a secondary memory 1110. The secondary memory 1110 may include, for example, a hard disk drive 1112 and/or a removable storage drive 1114, representing a floppy disk drive, a magnetic tape drive, an optical disk drive, etc. The removable storage drive 1114 reads from and/or writes to a removable storage unit 1118 in a well known manner. Removable storage unit 1118 represents a floppy disk, magnetic tape, optical disk, etc. which is read by and written to by removable storage drive 1114. As will be appreciated, the removable storage unit 1118 includes a computer usable storage medium having stored therein computer software and/or data.

In alternative embodiments, secondary memory 1110 may include other similar devices for allowing computer programs or other instructions to be loaded into computer system 1100. Such devices may include, for example, a removable storage unit 1122 and an interface 1120. Examples of such may include a program cartridge and cartridge interface (such as that found in video game devices), a removable memory chip (such as an erasable programmable read only memory (EPROM), or programmable read only memory (PROM)) and associated socket, and other removable storage units 1122 and interfaces 1120, which allow software and data to be transferred from the removable storage unit 1122 to computer system 1100.

Computer system 1100 may also include a communications interface 1124. Communications interface 1124 allows software and data to be transferred between computer system 1100 and external devices. Examples of communications interface 1124 may include a modem, a network interface (such as an Ethernet card), a communications port, a Personal Computer Memory Card International Association (PCMCIA) slot and card, etc. Software and data transferred via communications interface 1124 are in the form of signals 1128 which may be acoustic, ultrasonic, electronic, electromagnetic, optical or other signals capable of being received by communications interface 1124. These signals 1128 are provided to communications interface 1124 via a communications path (e.g., channel) 1126. This channel 1126 carries signals 1128 and may be implemented using wire or cable, fiber optics, a telephone line, a cellular link, a radio frequency (RF) link, an acoustic frequency link, an ultrasonic frequency link, and other communications channels.

In this document, the terms "computer program medium" and "computer usable medium" are used to generally refer to media such as removable storage drive 1114 and a hard disk installed in hard disk drive 1112. These computer program products provide software to computer system 1100.

Computer programs (also referred to as computer control logic) are stored in main memory 1108 and/or secondary memory 1110. Computer programs may also be received via communications interface 1124. Such computer programs, when executed, enable the computer system 1100 to perform the features of the present invention, as discussed herein. In particular, the computer programs, when executed, enable the processor 1104 to perform the features of the present invention. Accordingly, such computer programs represent controllers of the computer system 1100.

In an embodiment, the software may be stored in a computer program product and loaded into computer system 1100 using removable storage drive 1114, hard drive 1112 or communications interface 1124. The control logic (software), when executed by the processor 1104, causes the processor 1104 to perform the functions of the invention as described herein.

In another embodiment, the invention is implemented primarily in hardware using, for example, hardware components such as application specific integrated circuits (ASICs). Implementation of the hardware state machine so as to perform the functions described herein will be apparent to persons skilled in the relevant art(s).

In yet another embodiment, the invention is implemented using a combination of both hardware and software.

III. Locating A Swallowable Sensor Device Disposed Within A Patient In Accordance With An Embodiment.

Embodiments provide methods, systems, and apparatuses for locating a swallowable sensor device. Such methods, systems, and apparatuses may be used, for example, to locate the swallowable sensor device as it travels through a patient's gastrointestinal tract.

A. Positioning Of Sensor Link Modules On A Patient In Accordance With An Embodiment

To locate swallowable sensor device 104 as it travels through patient 102, a plurality of sensor link modules are positioned on patient 102. In an embodiment, the plurality of sensor link modules are positioned on a front portion of patient 102. Positioning the plurality of sensor link modules on the front portion of patient 102 may reduce distortions (such as multi-path distortions) caused by bones included on the back portion of patient 102. In another embodiment, the plurality of sensor link modules include four, five, or more sensor link modules. Increasing the number of sensor link modules that are used may increase the accuracy in locating swallowable sensor device 104. In a further embodiment, each sensor link module includes a plurality of sensing elements. For example, each sensor link module may include a plurality of sensing elements oriented in a hexagonal close pack configuration as illustrated in FIG. 8. Each sensing element may convert a received acoustic signal into an electric signal, which is detectable by a detector (e.g., charge coupled device (CCD) or direct conversion receiver).

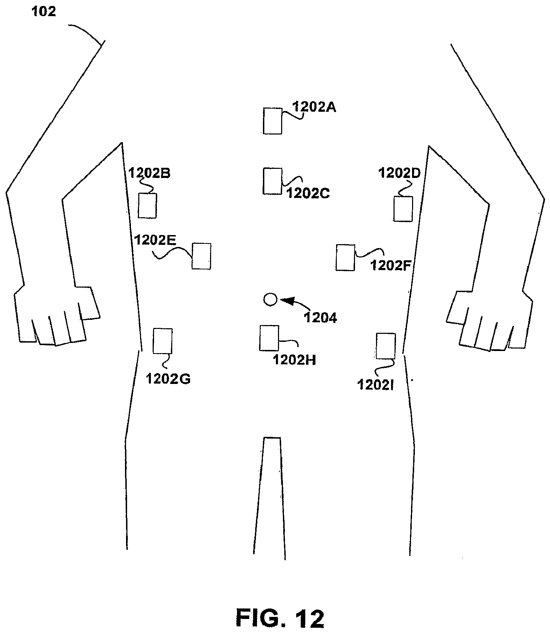

FIG. 12 depicts an embodiment in which nine sensor link modules 1202A-I are positioned on the front of patient 102. The navel 1204 of patient 102 is used as a reference point about which sensor link modules 1202A-I are positioned. The location of each sensor link module 1202 can be determined with respect to the other sensor link modules and a reference point using known techniques - such as techniques described in U.S. Patent No. 7,160,258 to Imran et al. Once the location of sensor link modules 1202 is known, the location of swallowable sensor device 104 can be determined, as described in more detail below.

It is to be appreciated, however, that the positioning of sensor link modules 1202A-I depicted in FIG. 12 is for illustrative purposes only, and not limitation. Other orientations of sensor link modules may be realized. Given the plurality of sensor link modules positioned on a front of patient 102, several different types of locating methods and calculations may be performed in accordance with embodiments, as described in more detail below.

B. An Example Locating Method

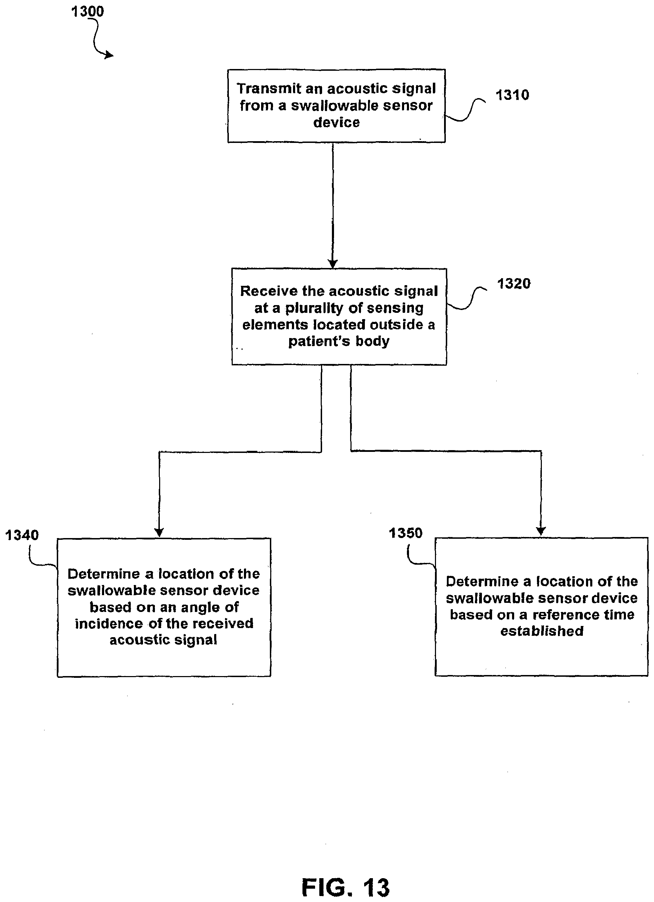

FIG. 13 depicts a block diagram of an example method 1300 for locating swallowable sensor device 104 disposed within patient 102. Method 1300 begins at a step 1310 in which an acoustic signal, such as signal 106, is transmitted from swallowable sensor device 104.

In a step 1320, the acoustic signal transmitted by swallowable sensor device 104 is received by a plurality of sensing elements located outside the body of patient 102. The plurality of sensing elements may be located on one or more sensor link modules (such as sensor link modules 1202) positioned on patient 102. In an embodiment, the time at which the acoustic signal is received at each of sensing elements is determined. For examples, the arrival time may be determined based on the phase of a counter included in each sensor link module, a signal strength indicator circuit, the output of a finite impulse response (FIR) filter, or the like.

In an embodiment, as illustrated in a step 1340, the location of swallowable sensor device 104 is determined based on an angle of incidence of the acoustic signal received by at least a subset of sensing elements. In this embodiment, the plurality of sensing elements comprise a phased array of sensing elements. A computation module (such as control logic 702 included on sensor link module 602, control logic 214 included in swallowable sensor module 104, or other control logic) computes the location of swallowable sensor device 104 based on the acoustic signal received by at least a subset of sensing elements, as set forth in more detail below.

In an alternative embodiment, as illustrated in a step 1350, the location of swallowable sensor device 104 is determined based on a reference time and a time difference of arrival of an acoustic signal received by ones of the plurality of sensing elements. The reference time is established when a given sensing element receives the acoustic signal, wherein the given sensing element receives the acoustic signal before at least a subset of the other sensing elements. For example, the reference time can be established when the sensing element closest to the swallowable sensor device receives the acoustic signal. A person skilled in the relevant art(s) will appreciate that the sensing element closest to the swallowable sensor device will be the first sensing element to receive the acoustic signal. Based on the reference time and time difference of arrival, a computation module (such as control logic 702 included on sensor link module 602, control logic 214 included in swallowable sensor module 104, or other control logic) computes the location of swallowable sensor device 104, as set forth in more detail below.

Example calculations that may be used to determine the location of swallowable sensor device 104 in accordance with the phased array embodiment depicted in step 1340 and the time difference of arrival embodiment depicted in step 1350 are set forth below in Section C and D, respectively.

C. Example Calculations To Determine The Location Of A Swallowable Sensor Device Using Phased Array Sensing Elements

In an embodiment, the location of swallowable sensor device 104 can be determined based on the time that at least a subset of the sensing elements receive the signal transmitted by swallowable sensor device 104. In this example, the sensing elements comprise a phased array, as described in more detail below. Described below is a two-dimensional example for locating swallowable sensor device 104. This example can be extended to three dimensions as would be apparent to a person skilled in the relevant art(s) from reading the description contained herein. It is to be appreciated that two- and three-dimensional locating methods and systems are within the spirit and scope of the present description. To better illustrate calculations that can be used to locate a swallowable sensor device, the example calculations presented below assume that the body of a patient is a homogenous medium, such that the speed of sound is the same throughout the patient's entire body. A person skilled in the relevant art(s) will appreciate, however, that the human body is not a homogenous medium. The speed of sound may be different in different types of body tissue (such as a kidney, a liver, a heart, etc.) and different types of body structures (such as bone, cartilage, etc.). It is to be appreciated that the example calculations presented below are for illustrative purposes only, and not limitation.

The speed of sound in body tissue, referred to herein as $c_b$, is approximately 1540 meters per second. The speed of sound in a body tissue is related to the frequency and wavelength of the sound by the well-known equation

$$c = \lambda f \qquad\qquad (\text{Eq. 1})$$

where $c$ is the speed of sound in body tissue, $\lambda$ is the wavelength of the sound in the body tissue, and $f$ is the frequency of the sound in the medium. Thus, a sound wave with a frequency of 1 megahertz propagating in body tissue with a speed of sound of 1540 meters per second will have a wavelength of approximately 1.54 millimeters, in accordance with the Eq. 1.

Swallowable sensor device 104 transmit acoustic signal 106 that radiates outward in multiple directions. Due to the finite speed of sound, acoustic signal 106 transmitted by swallowable sensor device 104 will take a finite amount of time to reach the sensing elements of sensor link modules 1202. Due to the location of swallowable sensor device 104 with respect to sensor link modules 1202, acoustic signal 106 may traverse different paths to reach ones of the sensing elements of sensor link module 1202. Thus, acoustic signal 106 may arrive at the sensing elements at different times.

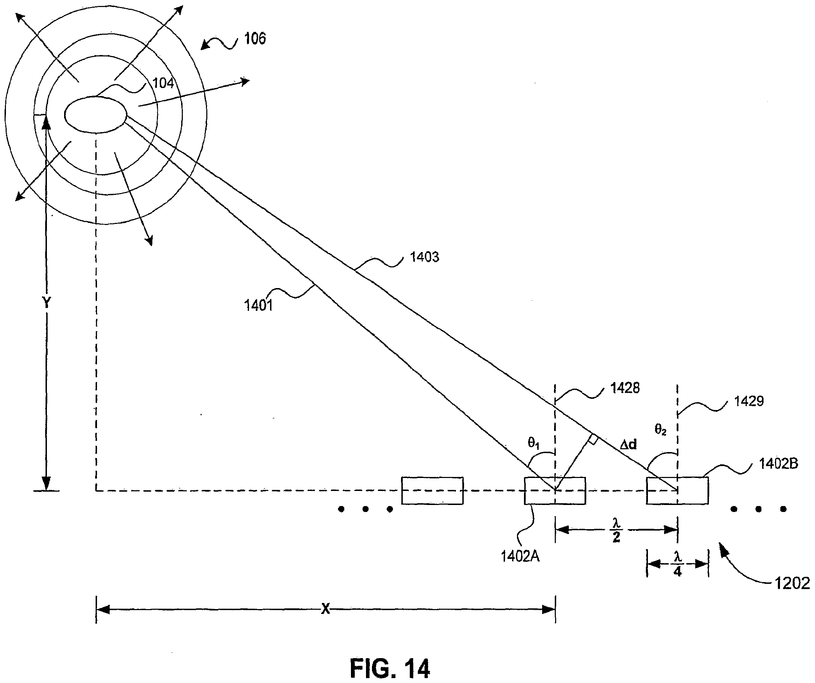

For example, FIG. 14 depicts an example location of swallowable sensor device 104 with respect to sensor link module 1202. As illustrated in FIG. 14, swallowable sensor device 104 transmits acoustic signal 106, which radiates outward from swallowable sensor device 104 in multiple directions. Signal 106 transmitted by swallowable sensor device 104 traverses a first path 1401 to reach a first sensing element 1402a, and impinges on first sensing element 1402a at an angle $\theta_1$ with respect to normal 1428. Similarly, signal 106 transmitted by swallowable sensor device 104 traverses a second path 1403 to reach a second sensing element 1402b of sensor link module 1202, and impinges on second sensing element 1402b at an angle $\theta_2$ with respect to normal 1429.

Due to the location of swallowable sensor device 104 with respect to sensor link module 1202 in the example depicted in FIG. 14, a length $d_2$ of second path 1403 is greater than a length $d_1$ of first path 1401. The difference in lengths, referred to herein as the path difference $\Delta d$, is given by

$$\Delta d = d_2 - d_1. \qquad \text{(Eq. 2)}$$

More generally, the path difference between any two successive sensing elements is given by

where $i$ is an index that serves as a label for sensing elements and can be any natural number, whole number, or integer number, as would be apparent to a person skilled in the relevant art(s).

In an embodiment, each sensing element 1402 has a width of approximately $\lambda/4$ and a center-to-center separation between successive sensing elements of approximately $\lambda/2$. In this embodiment, sensing elements 1402 comprise a phased array because sensing elements 1402 are separated from each other by a predetermined fraction of the wavelength of acoustic signal 106. Sensing elements 1402 of the phased array may be disposed on a single sensor link module 1202 (as depicted in FIG. 14) or may be disposed on different sensor link modules.

For a phased array, the maximum path difference - *i.e.,* time delay - occurs when swallowable sensor device 104 is edge on with sensing elements 1402 of sensor link module 1202. For example, at a sound frequency of 385 KHz, the maximum time delay is given by

$$\tau_{\max} = \frac{\lambda/2}{c} \approx 1.3\,\mu\sec \qquad \text{(Eq. 4)}$$

where $\tau_{\max}$ is the maximum time delay between successive sensing elements 1402. Because acoustic signal 106 takes a finite amount of time to propagate through body tissue, the difference in time that it takes acoustic signal 106 to reach successive sensing elements can be used to determined the location of swallowable sensor device 104 disposed in a body.

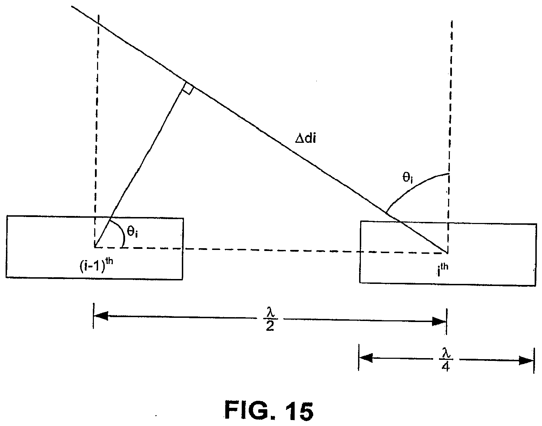

FIG. 15 illustrates a close up view of an example geometry depicting the path difference $\Delta d_i$ between two successive sensing elements, labeled as the $i^{th}$ and the $(i\text{-}1)^{th}$ sensing elements, wherein $i$ is an index representing any integer number. Based on the geometry depicted in FIG. 15, the path difference $\Delta d_i$ is given by

$$\Delta d_i = \frac{\lambda}{2}\sin(\theta_i) \qquad \text{(Eq. 5)}$$

where $\lambda$ is the wavelength of acoustic signal 106 and $\theta_i$ is the angle that acoustic signal 106 makes with the normal.

Eq. 5 can be rearranged in the following manner

$$\theta_i = \sin^{-1}\left(\frac{2\Delta d_i}{\lambda}\right). \qquad \text{(Eq. 6)}$$

The path difference $\Delta d_i$ can also be expressed as

$$\Delta d_i = c_b \cdot \tau_i \qquad \text{(Eq. 7)}$$

where $c_b$ is the speed of propagation in the medium (e.g., the body) and $\tau_i$ is the amount of time that the propagating signal takes to traverse the distance $\Delta d_i$.

Substituting the expression for $\Delta d_i$ from Eq. 7 into Eq. 6 yields

$$\theta_i = \sin^{-1}\left(\frac{2c\tau_i}{\lambda}\right). \qquad {}_8 \qquad\qquad \text{(Eq. 8)}$$

Each sensing element 1402 may include or be coupled to a counter that has a clock phase given by $\varphi$. Based on such a clock phase, the time, $\tau_i$, at which acoustic signal 106 is received at the i-th sensing element can be determined in the following manner

$$N_i = \varphi\tau_i \Rightarrow \tau_i = \frac{N_i}{\varphi}. \qquad\qquad \text{(Eq. 9)}$$

Substituting the expression for the arrival time, given in Eq. 9, into Eq. 8 yields

$$\theta_i = \sin^{-1}\left(\frac{2cN_i}{\lambda\varphi}\right). \qquad\qquad \text{(Eq. 10)}$$

Referring back to the geometry depicted in FIG. 14, the horizontal distance $x$ and the vertical distance $y$ from swallowable sensor device 104 to first sensing element 1402a can be related to the angle of incidence $\theta_1$ by the following equation:

$$\frac{x}{y} = \tan\theta_1. \qquad\qquad \text{(Eq. 11)}$$

Substituting the expression for $\theta_1$ given by Eq. 10 into Eq. 11 yields

$$\frac{x}{y} = \tan\left[\sin^{-1}\left(\frac{2c_b N_1}{\lambda\varphi}\right)\right]. \qquad\qquad \text{(Eq. 12)}$$

Eq. 12 expresses the location of swallowable sensor device 104 with respect to first sensing element 1402a in terms of the time that signal 106 arrives at first sensing element 1402a.
In a similar manner, the location of swallowable sensor device 104 with respect to second sensing element 1402b can be expressed in terms of the time that signal 106 arrives at second sensing element 1402b, as follows:

$$\frac{x + \lambda/2}{y} = \tan\left[\sin^{-1}\left(\frac{2c_b N_2}{\lambda\varphi}\right)\right]. \qquad\qquad \text{(Eq 13)}$$

The location of swallowable sensor device 104 can then be determined from Eq. 12 and Eq. 13 because there are two unknowns (namely, $x$ and $y$) and two equations.
In accordance with the two-dimensional example presented above, a minimum of two phased-array sensing elements is required to locate swallowable sensor device 104. In three-dimensional example, a minimum of three phased-array sensing elements is required to locate swallowable sensor device 104. For example, each sensing element may be located on sensor link module 1202, so that sensor link modules 1202 comprise a phased array.
It is to be appreciated, however, that a greater number of phased-array sensing elements may be used. Using a greater number of phased-array sensing elements provides redundancy to more accurately determine the location of swallowable sensor device 104. For example, more than three sensor link module 1202 may be positioned on patient 102 in a phased array. Additionally or alternatively, each sensor link module 1202 may include a large number of sensing elements, such as tens, hundreds or thousands of sensing elements.

D. Example Calculations To Determine The Location Of A Swallowable Sensor Device Using Single Element Receivers

In an embodiment, the location of swallowable sensor device 104 can be determined based on a reference time and a time difference of arrival of an acoustic signal received by sensor link modules 1202 positioned on patient 102. The reference time may be established based on a time when a first sensing element receives acoustic signal 106 transmitted by swallowable sensor device 104, wherein the first sensing element receives acoustic signal 106 before at least a subset of the other sensing elements. For example, the reference time can be established when the sensing element closest to swallowable sensor device 104 receives the acoustic signal transmitted by swallowable sensor device 104. Set forth below are example calculations for determining the location of swallowable sensor device 104 in accordance with this embodiment.

i. A First Set Of Example Calculations

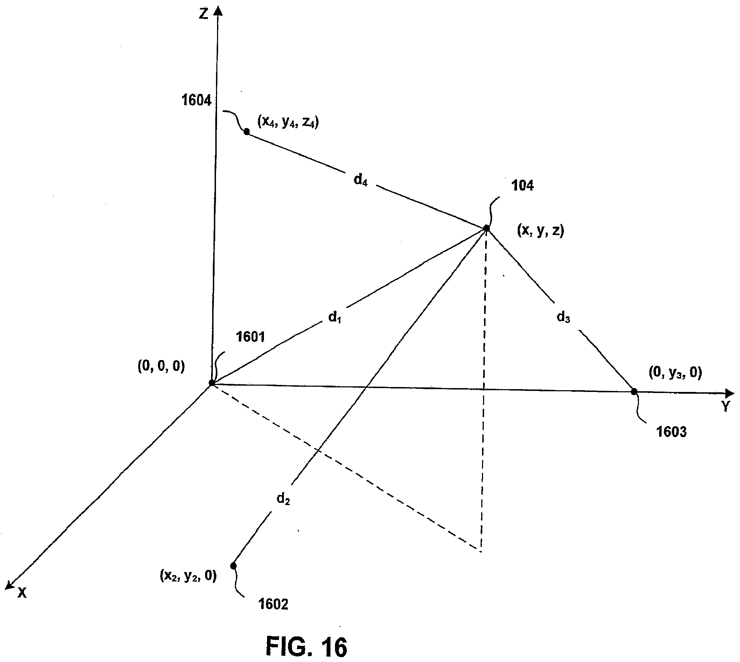

In an embodiment, at least four sensing elements are used to determine the location of swallowable sensor device 104. For example, FIG. 16 illustrates a Cartesian coordinate system for determining the location of swallowable sensor device 104. The at least four sensing elements that receive an acoustic signal transmitted by swallowable sensor device 104 are illustrated in FIG. 16 as a first sensing element 1601, a second sensing element 1602, a third sensing element 1603, and a fourth sensing element 1604.

With respect to the Cartesian coordinate system of FIG. 16, first sensing element 1601 is located at $(0, 0, 0)$, second sensing element 1602 is located at $(x_2, y_2, 0)$, third sensing element 1603 is located at $(0, y_3, 0)$, fourth sensing element 1604 is located at $(x_4, y_4, z_4)$, and swallowable sensor device 104 is located at $(x, y, z)$. The location of the sensing elements can be determined using known techniques, such as, for example, techniques described in U.S. Patent No. 7,160,258 to Imran et al.. Thus, with respect to the equations set forth below, the coordinates $(0, 0, 0)$, $(x_2, y_2, 0)$, $(0, y_3, 0)$, and $(x_4, y_4, z_4)$ - *i.e.,* the locations of the four sensing elements 1601, 1602, 1603, and 1604 - represent known quantities. In contrast, the coordinates $(x, y, z)$ - *i.e.,* the location of swallowable sensor device 104 - represent unknown quantities.

As illustrated in FIG. 16, the four sensing elements 1601, 1602, 1603, and 1604 are separated from swallowable sensor device 104 by distances $d_1, d_2, d_3,$ and $d_4$, respectively. The distances $d_i$ are given by the following general equation

$$d_i = \sqrt{(x-x_i)^2 + (y-y_i)^2 + (z-z_i)^2} \; , \qquad \text{(Eq. 14)}$$

wherein *i* is an index running from 1 to 4, $(x, y, z)$ are the coordinates of swallowable sensor device 104, and $(x_i, y_i, z_i)$ are the coordinates of the i-th sensing element. Thus, in terms of the example coordinates given above, the distances $d_1, d_2, d_3,$ and $d_4$ are given by the following equations:

$$d_1 = \sqrt{x^2 + y^2 + z^2} \qquad \text{(Eq. 15a)}$$

$$d_2 = \sqrt{(x-x_2)^2 + (y-y_2)^2 + z^2} \qquad \text{(Eq. 15b)}$$

$$d_3 = \sqrt{x^2 + (y-y_3)^2 + z^2} \qquad \text{(Eq. 15c)}$$

$$d_4 = \sqrt{(x-x_4)^2 + (y-y_4)^2 + (z-z_4)^2} \qquad \text{(Eq. 15d)}$$

For illustrative purposes, and not limitation, the discussion below assumes that these distances are not equal to each other and that $d_1 < d_2 < d_3 < d_4$.

Because the distances $d_i$ are not equal to each other, the acoustic signal will arrive at each of the sensing elements at different times. First sensing element 1601 will receive the acoustic signal at time $t_1$, second sensing element 1602 will receive the acoustic signal at time $t_2$, third sensing element 1603 will receive the acoustic signal at time $t_3$, and fourth sensing element 1604 will receive the acoustic signal at time $t_4$.

The time, $t_1$, is used as a reference time for determining the location of swallowable sensor device 104. The difference between the reference time, $t_1$, and the time that the acoustic signal arrives at the other sensing elements can be measured. These time differences can be used in the following equations:

$$d_2 - d_1 = c \cdot \Delta t_{12} \qquad \text{(Eq. 16a)}$$

$$d_3 - d_1 = c \cdot \Delta t_{13} \qquad \text{(Eq. 16b)}$$

$$d_4 - d_1 = c \cdot \Delta t_{14} \qquad \text{(Eq. 16c)}$$

wherein c is the speed of sound in a patient's body (which is approximately 1540 m/s), $\Delta t_{12}$ is the difference between $t_2$ and $t_1$, $\Delta t_{13}$ is the difference between $t_3$ and $t_1$, and $\Delta t_{14}$ is the difference between $t_4$ and $t_1$.

Inserting the expressions for the distances $d_i$, given in Eqs. 15a-d, into Eqs. 16a-c yields

$$\sqrt{(x-x_2)^2 + (y-y_2)^2 + z^2} - \sqrt{x^2 + y^2 + z^2} = c \cdot \Delta t_{12} \qquad \text{(Eq. 17a)}$$

$$\sqrt{x^2 + (y-y_3)^2 + z^2} - \sqrt{x^2 + y^2 + z^2} = c \cdot \Delta t_{13} \qquad \text{(Eq. 17b)}$$

$$\sqrt{(x-x_4)^2 + (y-y_4)^2 + (z-z_4)^2} - \sqrt{x^2 + y^2 + z^2} = c \cdot \Delta t_{14} \qquad \text{(Eq. 17c)}$$

Because there are three equations and three unknowns (namely, the coordinates $(x, y, z)$), these equations can be used to determine the location of swallowable sensor device 104. For example, Eqs. 17a-c can be solved by using known techniques for solving systems of equations, as would be apparent to a person skilled in the relevant art(s).

ii. A Second Example Set Of Calculations

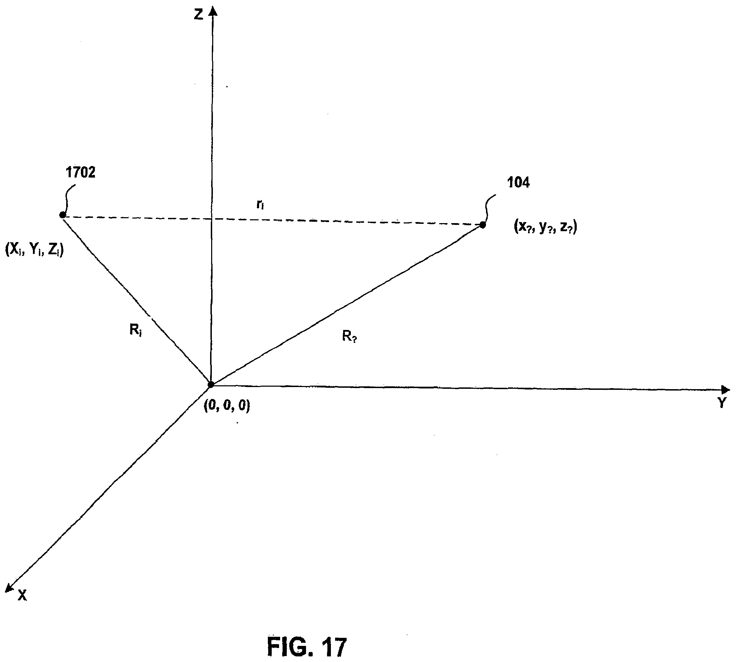

In another embodiment, at least five sensing elements are used to determine the location of swallowable sensor device 104. For example, FIG. 17 illustrates an example coordinate system for determining the location of swallowable sensor device 104.

Referring to FIG. 17, the at least five sensing elements 1702 have coordinates $(X_i, Y_i, Z_i)$, wherein $i$ is an integer index running from 1 to 5. The coordinates of sensing elements 1702 represent known quantities. The distance that sensing elements 1702 are from the origin of the coordinate system in FIG. 17 is given by the following equation:

$$R_i = \sqrt{X_i^2 + Y_i^2 + Z_i^2} \qquad \text{(Eq. 18)}$$

As illustrated in FIG. 17, swallowable sensor device 104 has coordinates $(x_?, y_?, z_?)$. The coordinates of swallowable sensor device 104 represent unknown quantities. The distance that swallowable sensor device 104 is from the origin is given by

$$R_? = \sqrt{x_?^2 + y_?^2 + z_?^2} \; . \qquad \text{(Eq. 19)}$$

The distance between the i-th sensing elements 1702 and swallowable sensor device 104 can be expressed in terms of the time that it takes an acoustic signal to travel from swallowable sensor device 104 to the i-th sensing element 1702:

$$r_i = c \cdot t_i \qquad \text{(Eq. 20)}$$

wherein c is the speed of sound in patient 102. The time $t_i$ can be used as a reference time as described above. The difference in the time of arrival between the acoustic signal received by the i-th sensing element 1702 and the j-th sensing element 1702 can be related to the difference in the distance between swallowable sensor device 104 and the i-th and j-th sensors 1702, respectively, in the following manner:

$$r_i - r_j = c \cdot (t_i - t_j) \equiv c \cdot \Delta t_{ij} \qquad \text{(Eq. 21)}$$

The distance between the i-th sensing element 1702 and swallowable sensing element 104 can also be expressed as

$$r_i^2 = (x_? - X_i)^2 + (y_? - Y_i)^2 + (z_? - Z_i)^2 \qquad \text{(Eq. 22)}$$

For the i-th and the j-th sensing elements, Eq. 22 can be recast in the following manner:

$$r_i^2 = r_?^2 - 2(X_i x_? + Y_i y_? + Z_i z_?) + R_i^2 \qquad \text{(Eq. 23a)}$$

$$r_j^2 = r_?^2 - 2(X_j x_? + Y_j y_? + Z_j z_?) + R_j^2 \qquad \text{(Eq. 23b)}$$

wherein $r_?^2 = x_?^2 + y_?^2 + z_?^2$ and $R_i^2 = X_i^2 + Y_i^2 + Z_i^2$.

Subtracting Eq. 23b from Eq. 23a yields the following equation:

$$r_i^2 - r_j^2 = -2((X_i - X_j)x_? + (Y_i - Y_j)y_? + (Z_i - Z_j)z_?) + R_i^2 - R_j^2 \qquad \text{(Eq. 24)}$$

The difference $r_i^2 - r_j^2$ can be factored and then Eq. 21 can be used to recast the left side of Eq. 24 in the following manner:

$$r_i^2 - r_j^2 = (r_i + r_j) \cdot (r_i - r_j) = (r_i + r_j) \cdot c \cdot (t_i - t_j) \qquad \text{(Eq. 25)}$$

Substituting the result from Eq. 25 into the left side of Eq. 24 yields the following result:

$$(r_i + r_i) \cdot c \cdot \Delta t_{ij} = -2((X_i - X_j)x_? + (Y_i - Y_j)y_? + (Z_i - Z_j)z_?) + R_i^2 - R_j^2 \qquad \text{(Eq. 26a)}$$

A similar expression can be written for the j-th and the k-th sensing elements:

$$(r_j + r_k) \cdot c \cdot \Delta t_{jk} = -2((X_j - X_k)x_? + (Y_j - Y_k)y_? + (Z_j - Z_k)z_?) + R_j^2 - R_k^2 \qquad \text{(Eq. 26b)}$$

Multiplying Eq. 26a by $\Delta t_{jk}$ and Eq. 26b by $\Delta t_{ij}$, and subtracting the resulting expressions yields the following result:

$$\begin{aligned}
c\Delta t_{ik}\Delta t_{ij}\Delta t_{jk} = &-2[(X_j - X_k)\Delta t_{ij} + (X_i - X_j)\Delta t_{jk}]x_? \\
&-2[(Y_j - Y_k)\Delta t_{ij} + (Y_i - Y_j)\Delta t_{jk}]y_? \\
&-2[(Z_j - Z_k)\Delta t_{ij} + (Z_i - Z_j)\Delta t_{jk}]z_? \\
&+(R_i^2 - R_j^2)\Delta t_{jk} - (R_j^2 - R_k^2)\Delta t_{ij}
\end{aligned} \qquad \text{(Eq. 27)}$$

By allowing the indices *i, j, k* to run from 1 to 5, Eq. 27 represents 10 linearly independent equations. These 10 linearly independent equations can be solved in terms of the coordinates $(x_?, y_?, z_?)$ of swallowable sensor device 104. Thus, the position of swallowable sensor device 104 can be determined from Eq. 27.

iii. Summary Of The First And Second Example Set Of Calculations

Because the above-described methods establish a reference time without requiring swallowable sensor device 104 to transmit a separate type of reference signal - such as an RF signal or other type of electromagnetic (EM) signal - the above-described methods have several example advantages. As a first example, swallowable sensor device 104 can be smaller and less complicated compared to a device that includes an RF signal generator. In addition, the above-described methods advantageously do not require the use of an EM signal, which may be attractive to potential patients since the extent to which EM signals harm body tissue is not fully known at this time.

In accordance with the example equations presented above, a minimum of four or five sensing elements are used to locate swallowable sensor device 104. In an embodiment, however, a greater number of sensing elements can be used to determine the location of swallowable sensor device 104. Using a greater number provides redundancy to more accurately determine the location of swallowable sensor device 104. For example, a plurality of sensing elements can be disposed on each sensor link module 1202, and multiple sensor link modules 1202 can be caused to adhere to different portions of a patient's body - as illustrated, for example, in FIG. 12. Additionally or alternatively, a plurality of sensing elements can be disposed at a plurality of locations on a wearable fabric that is worn by a patient. Also, the accuracy of the location calculations can be increased by using a combination of the phased array approach with the time based approached.

IV. Internal Imaging In Accordance With An Embodiment

Swallowable sensor device 104 may be used to image an internal portion of a patient, such as a portion of or an object included in the patient's gastrointestinal tract. Example methods for imaging an internal portion of a patient are set forth below.

A. Example Methods For Internally Imaging A Patient

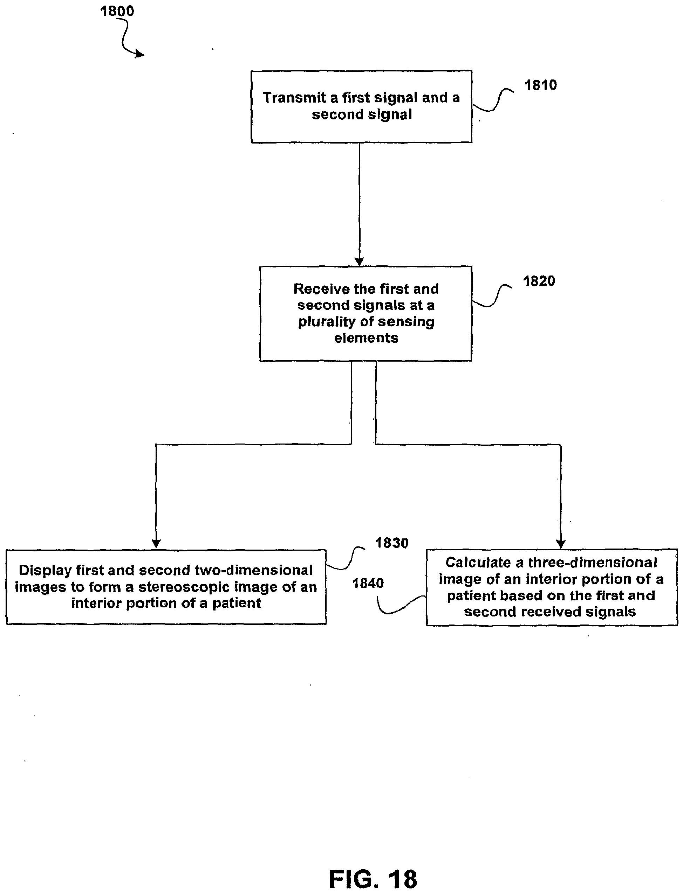

FIG. 18 depicts a block diagram illustrating an example method 1800 for imaging an internal portion of patient 102 - such as a portion of the patient's gastrointestinal tract, a tumor included in the gastrointestinal tract, a fetus, or some other interior portion of the patient.

Method 1800 begins at a step 1810 in which a first acoustic signal is transmitted from a first location and a second acoustic signal is transmitted from a second location. The first and second acoustic signals may be transmitted by swallowable sensor device 104 as it travels through a patient's gastrointestinal tract. Additionally or alternatively, the first and second acoustic signals may be transmitted by one or more devices external to patient 102, such as one or more external computing devices 108, one or more sensor link modules 602, an electronic fabric worn by patient 102 that comprises a plurality of acoustic transducer elements, or one or more other external devices as would be apparent to a person skilled in the relevant art(s).

In a step 1820, the first and second acoustic signals are received by a plurality of sensing elements. The plurality of sensing elements may be included in one or more swallowable sensor devices. Additionally or alternatively, the plurality of sensing elements may be included in one or more devices external to patient 102, such as one or more external computing devices 108, one or more sensor link modules 602 or 1202, an electronic fabric worn by patient 102 that comprises a plurality of acoustic transducer elements, or one or more other external devices as would be apparent to a person skilled in the relevant art(s). The sensing elements may include, for example, a transducer (such as transducer 902) for determining the amplitude of the received signal at each of the plurality of sensing elements, and may be coupled to a counter (such as counter 908) for determining a time at which the first and second acoustic signals are respectively received at each of the plurality of sensing elements.

In a first embodiment, as illustrated in step 1830, a stereoscopic image is generated based on the first and second acoustic signals received by the plurality of sensing elements. In this embodiment, the plurality of sensing elements capture two two-dimensional images: (1) a first two-dimensional image of an interior portion of patient 102 based on the first received acoustic signal; and (2) a second two-dimensional image of the interior portion of patient 102 based on the second received acoustic signal. The first and second two-dimensional images are stereoscopically displayed to form a three-dimensional image of the interior portion of patient 102. The stereoscopic display may be performed by a display device that is coupled to a device external to patient 102, such as, for example, external computing device 108 or remote entity 502. Example methods for capturing two two-dimensional images are described in more detail below in, for example, Section B.

In a second embodiment, as illustrated in a step 1840, a three-dimensional image of an interior portion of patient 102 is calculated based on the first and second received signals. Such three-dimensional imaging includes the calculation of three-dimensional volume elements, or "voxels." In an example, the three-dimensional image is calculated by a computation module. The computation module may be included in control logic stored in swallowable sensor device 104 and/or in an external device such as, for example, external computing device 108, sensor link module 602, and/or another device. Example equations for calculating voxels are described in more detail below in, for example, Section C.

B. Image Capture For Three Dimensional Viewing

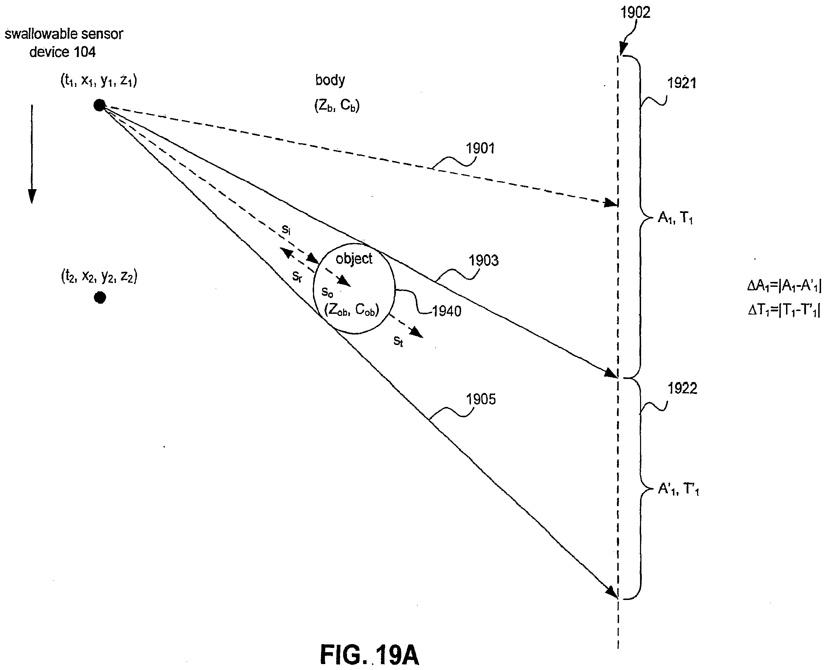

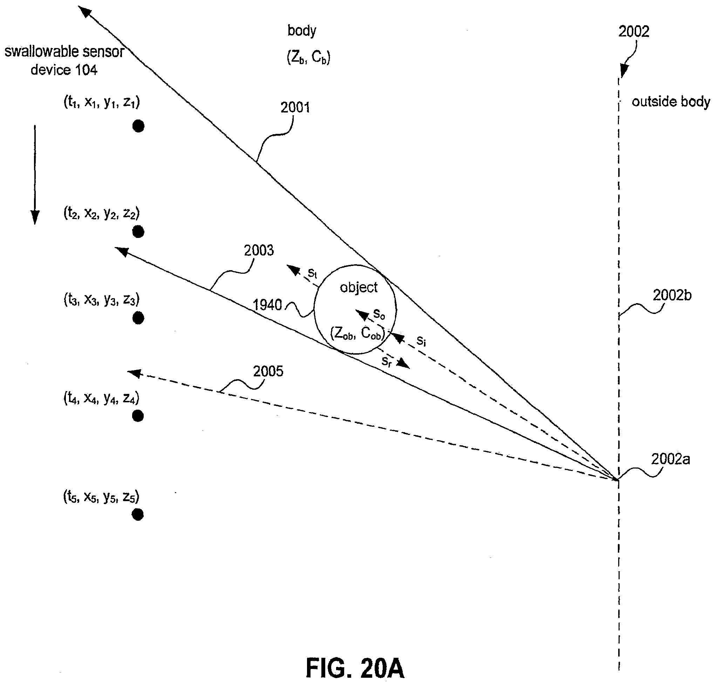

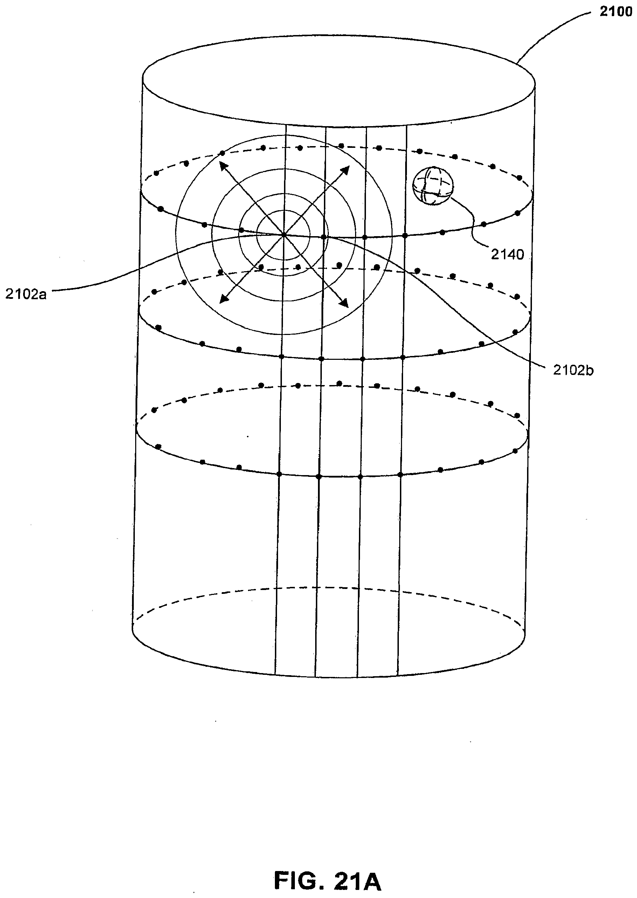

As set forth above in step 1830 (FIG. 18), a stereoscopic image of an interior portion can be generated based on two two-dimensional images of the interior portion of patient 102. In embodiments, the stereoscopic image is based on: (1) "shadow" images formed from acoustic signals transmitted by swallowable sensor device 104 and received by an external entity (FIGS. 19A-B); (2) reflective images formed from acoustic signals transmitted by swallowable sensor device 104 and received by one or more swallowable sensor devices 104 (FIGS. 19A-B); (3) "shadow" images formed from acoustic signals transmitted by an external entity and received by one or more swallowable sensor devices 104 (FIGS. 20A-B); (4) reflective images formed from acoustic signals transmitted by an external entity and received by the external entity (FIGS. 21A-B); and (5) "shadow" images formed from acoustics signals transmitted by an external entity and received by the external entity (FIGS. 21A-B). Each of these embodiments is described in more detail below.

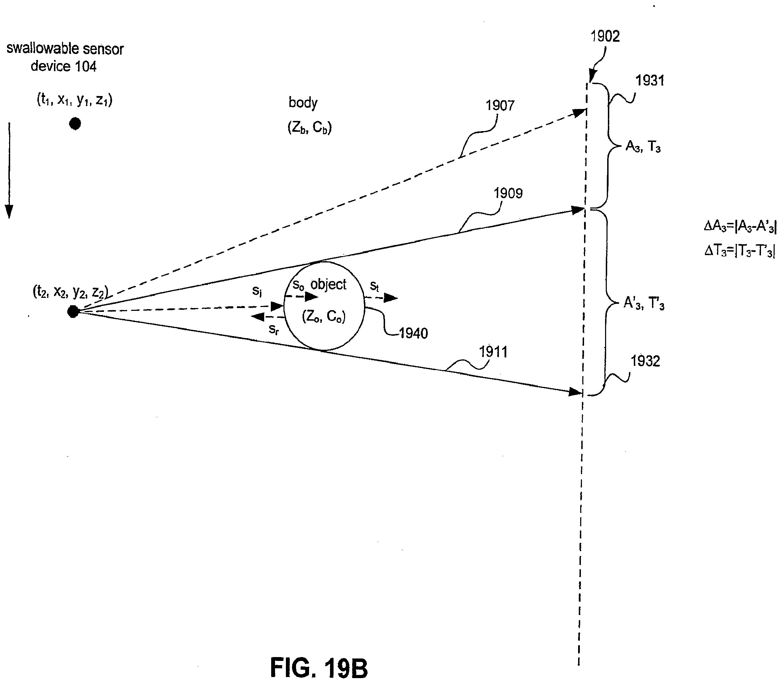

FIGS. 19A and 19B illustrate an example method for imaging an interior portion of patient 102 based on a signal transmitted by swallowable sensor device 104 according to an embodiment. For illustrative purposes, and not limitation, the example method illustrates the imaging of an object 1940 included in patient 102. Object 1940 has a characteristic impedance $Z_{ob}$, which is different than the characteristic impedance $Z_b$ of the patient's body. Also, the signal transmitted by swallowable sensor device 104 travels at a characteristic speed $C_{ob}$ as it traverses object 1940 and at a characteristic speed $C_b$ as it traverses the patient's body.

Included in each of FIGS. 19A and 19B is swallowable sensor device 104 and external sensing elements 1902. External sensing elements 1902 may be included, for example, on one or more sensor link modules 1202 (FIG. 12). Swallowable sensor device 104 is illustrated at different times and locations as it travels through patient 102. Swallowable sensor device 104 is configured to transmit acoustic signals 106 at the different times and locations. The different locations of swallowable sensor device 104 can be determined, as set forth above. As described in more detail below, because the different locations can be determined, the transmitted acoustic signal 106 received by external sensing elements 1902 can be used to generate an image of object 1940, referred to herein as a "shadow" image. Additionally or alternatively, the transmitted acoustic signal 106 may be received by sensing elements included in swallowable sensor device 104 after it reflects off object 1940 to generate an image of object 1940, referred to herein as a "reflective" image.

The generation of a "shadow" image is now described. Referring to FIG. 19A, at a first time $t_1$ corresponding to a first location $(x_1, y_1, z_1)$, swallowable sensor device 104 can transmit acoustic signal 106 that propagates outward in multiple directions. A transmitted acoustic signal traveling along a first path 1901 will not impinge on object 1940, but will directly impinge on a first collection 1921 of external sensing elements 1902. The acoustic signal received by sensing elements in the first collection 1921 has an amplitude $A_1$.

Unlike the transmitted acoustic signal traveling along first path 1901, transmitted acoustic signals traveling between a second path 1903 and a third path 1905, such as acoustic signal $S_i$, will impinge on object 1940. When the incident acoustic signal $S_i$ impinges on object 1940, a portion of incident acoustic signal $S_i$ will be reflected as a reflected acoustic signal $S_r$ and a portion of the incident acoustic signal $S_i$ will be transmitted through object 1940 as an acoustic signal $S_o$. The reflection occurs because object 1940 has a characteristic impedance $Z_{ob}$ that is different than the characteristic impedance $Z_b$ of the body. Similarly, after acoustic signal $S_o$ traverses object 1940 and impinges on the body, a portion of acoustic signal $S_o$ will be reflected and a portion will be transmitted. For clarity of presentation, only the transmitted portion of acoustic signal $S_o$, (namely, transmitted acoustic signal $S_t$) is illustrated in FIG. 19A. The reflection of acoustic signal $S_o$ is not shown.

Transmitted acoustic signal $S_t$ will then impinge on a second collection 1922 of external sensing elements 1902. The acoustic signal received by sensing elements in the second collection 1922 has an amplitude $A_1'$. Due to the reflection of the transmitted acoustic signal that impinged on object 1940, the amplitude $A_1'$ measured by the second collection 1922 of sensing elements will likely be different (e.g., less) than the amplitude $A_1$ measured by the first collection 1921 of sensing elements. That is, there will be a difference in amplitude $\Delta A$ equal to $A_1 - A_1'$.

The sensing elements in the first collection 1921 and the second collection 1922 can comprise or be coupled to a transducer that converts the received acoustic signal into an electric signal detectable by a detector (such as a charge coupled device (CCD) or a direct conversion receiver). Thus, the sensing elements can be used to capture a first "shadow" image that object 1940 casts when "illuminated" by transmitted signals traveling between second path 1903 and third path 1905. Because the location of swallowable sensor device 104 can be determined (as set forth above), the size of the "shadow" that object 1940 casts can be used to determine the size of object 1940 along a first dimension, such as a vertical dimension as illustrated in FIG. 19A.

A second "shadow" image of object 1940 may be generated by transmitting a second acoustic signal from swallowable sensor device 104 when it is at a second location $(x_2, y_2, z_2)$, as illustrated in FIGS. 19B. The second acoustic signal will propagate outward in multiple directions. Transmitted acoustic signals that impinge on a third collection 1931 of sensing elements, such as a transmitted acoustic signal traveling along a path 1907, will directly impinge on the third collection 1931 of external sensing elements 1902. The sensing elements in the third collection 1931 can determine an amplitude $A_3$ of the received acoustic signals. Similar to the second collection 1922 of sensing elements 1902, a fourth collection 1932 of sensing elements 1902 can detect a second "shadow" image that object 1940 casts when illuminated by transmitted signals traveling between path 1909 and path 1911, based on the amplitude $A_3'$ measured by the fourth collection 1932.

The first and second "shadow" images are two-dimensional images of object 1940 that will be slightly different because object 1940 was illuminated by acoustic signals that were transmitted from slightly different locations. The first and second "shadow" images can be encoded and transmitted to an external display device, such as a display device coupled to external computing device 108. The display device can then stereoscopically display the first and second "shadow" images to form a three-dimensional image of object 1940.

The generation of a reflective image is now described. At first location $(x_1, y_1, z_1)$, swallowable sensor device 104 can transmit a first acoustic signal that propagates outward in multiple directions. Transmitted acoustic signals that reflect off of object 1940, such as acoustic signal $S_r$, can then be detected by one or more swallowable sensor devices 104. The reflected acoustic signals detected by the one or more swallowable sensor devices 104 can be used to capture a first two-dimensional image of object 1940. Then, at second location $(x_2, y_2, z_2)$, swallowable sensor device 104 can transmit a second acoustic signal that propagates outward in multiple directions. Transmitted signals that reflect off of object 1940, such as signal $S_r$, can then be detected by one or more swallowable sensor devices 104 to capture a second two-dimensional image of object 1940. The first and second two-dimensional images can be encoded and sent to external computing device 108 via acoustic signal 106. The first and second two-dimensional images can then be stereoscopically displayed to form a three-dimensional image.

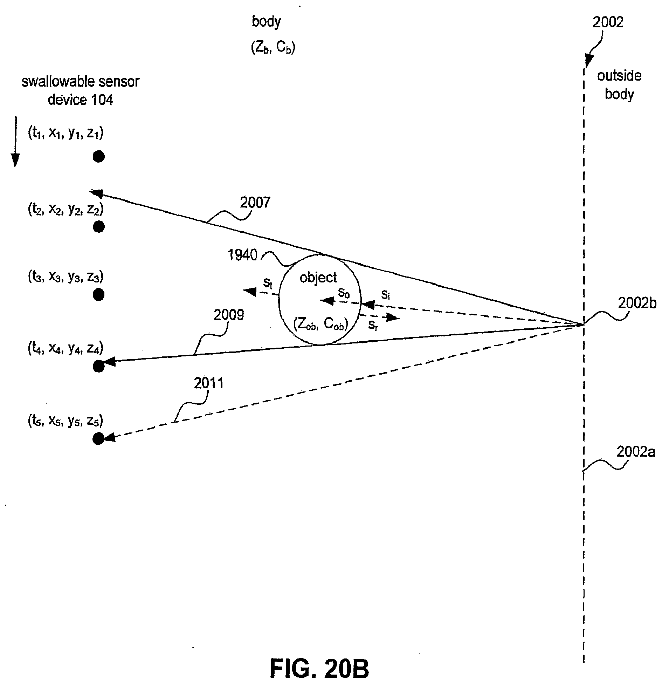

FIGS. 20A and 20B illustrate an example method for imaging an interior portion of patient 102 based on a plurality of signals transmitted from an external device to swallowable sensor device 104 according to an embodiment. For illustrative purposes, and not limitation, the example method illustrates the imaging of object 1940 included in patient 102.

Included in each of FIGS. 20A and 20B is swallowable sensor device 104 and a plurality of external elements 2002, including a first external element 2002a and a second external element 2002b. External elements 2002 may be acoustic transducer elements included on sensor link modules 1202, for example. Swallowable sensor device 104 is illustrated at different times and locations as it travels through patient 102. As illustrated in FIG. 20A, external elements can transmit acoustic signals that are detected by swallowable sensor device 104 to generate a stereoscopic "shadow" image of object 1940. Additionally or alternatively, the transmitted acoustic signal may be received by external elements 2002 after the transmitted acoustic signals reflect off object 1940 to generate a stereoscopic reflective image of object 1940.

Referring to FIG. 20A, first external element 2002a can transmit a first acoustic signal that propagates along multiple paths and that may be detected by swallowable sensor device 104 as it travels through patient 102. As swallowable sensor device 104 travels between path 2002 and 2003 it can capture a first "shadow" image that object 1940 casts when illuminated by the signals transmitted by first external element 2002a, in a similar manner to that described above. As swallowable sensor device continues traveling through patient 102, it can detect the full signal transmitted by first external element 2002a. For example, at a time between $t_3$ and $t_4$ swallowable sensor device 104 can detect a full signal transmitted by first external element 2002a that travels along path 2005.

Referring to FIG. 20B, second external element 2002b can transmit a second acoustic signal that propagates along multiple paths and that may be detected by swallowable sensor device 104 as it travels through patient 102. The second acoustic signal transmitted by second external element 2002b may have a different signature from the first acoustic signal transmitted by first external element 2002a, so that swallowable sensor device 104 may distinguish the first and second acoustic signals from each other. In a similar manner to that described above, as swallowable sensor device 104 travels between path 2007 and 2009 it can capture a second "shadow" image that object 1940 casts when illuminated by the acoustic signals transmitted by second external element 2002b. As swallowable sensor device continues traveling through patient 102, it can detect the full acoustic signal transmitted by second external element 2002b.

Because swallowable sensor device 104 can capture two two-dimensional "shadow" images of object 1940 as swallowable sensor device 104 travels through patient 102, a stereoscopic image of object 1940 can be formed. For example, swallowable sensor device 104 can encode the first and second "shadow" images and send these "shadow" images to an external device (such as external computing device 108 or sensor linking module 604). The first and second "shadow" images captured by swallowable sensor device 104 can then be stereoscopically displayed to form a three-dimensional image of object 1940.

Additionally or alternatively, the first and second acoustic signals transmitted by first and second external elements 2002a,b can be used to generate a stereoscopic reflective image of object 1940. A portion of the first acoustic signal transmitted by first external element 2002a will reflect off of object 1940 due to the impedance mismatch described above. These reflected signals, such as signal $S_r$, can then be detected by one or more of the external elements 2002 to capture a first two-dimensional image of object 1940. Similarly, a portion of the second acoustic signal transmitted by second external element 2002b will reflect off of object 1940. These reflected signals can then be detected by one or more of the external elements 2002 to capture a second two-dimensional image of object 1940. The first and second two-dimensional images can be encoded and sent to external computing device 108 for stereoscopic display, as described above.

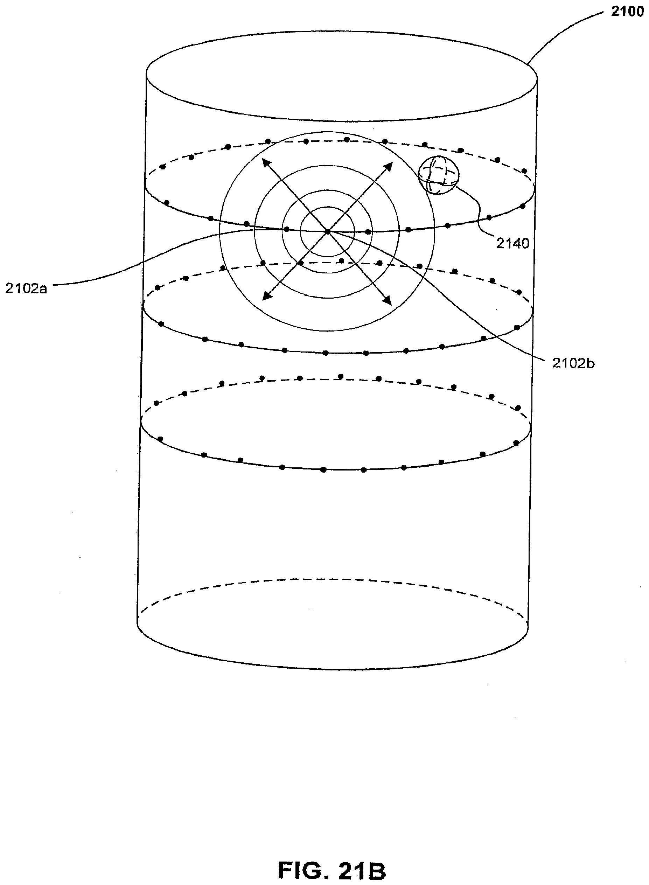

FIGS. 21A and 21B illustrate an array of sensing elements 2100 that is configured to encircle a patient (not shown) and generate "shadow" images of an interior portion of the patient in accordance with an embodiment. For example, array 2100 can be used to image an object 2140 included in the patient. Array 2100 includes a plurality of external elements that can transmit and receive acoustic signals, including a first external element 2102a and a second external element 2102b. In an example, the plurality of external elements may be configured on or in a wearable fabric that is worn by the patient. In an another example, the external elements may be included in one or more sensor link modules 1202 that are adhered to an exterior portion of the patient using an adhesive.

Referring to FIG. 21A, first external element 2102a can transmit a first acoustic signal at a first time. The first acoustic signal will propagate outward in multiple directions. The other external elements can then receive the first acoustic signal transmitted by first external element 2102a to capture a first "shadow" image of object 2140 in a similar manner to that described above.

Referring to FIG. 21B, second external element 2102b can transmit a second acoustic signal at a second time. The second acoustic signal will also propagate in multiple directions. The other external elements can then receive the second acoustic signal transmitted by second external element 2102b to capture a second "shadow" image of object 2140 in a similar manner to that described above. The first and second acoustic signals may have a different signature so that the external elements can distinguish the first and second acoustic signals.

The first and second "shadow" images captured by array 2100 can then be stereoscopically displayed to form a three-dimensional image of objective 2140.

In the methods described above, it is to be appreciated that more than two "shadow" images of an object can be captured. Capturing additional "shadow" images of an object can be used to provide multiple vantage points from which to view the object. Furthermore, the resolution of the captured images can be increased by increasing the number of sensing elements that capture the two-dimensional images.

In an embodiment, three-dimensional reflective images are obtained in a similar manner. In this embodiment, a first external sensor sends out a signal, and the other external sensors receive the reflected signal to form a first two-dimensional image. Likewise, a second external sensor sends out a signal, and the other external sensors receive the reflected signal to form a second two-dimensional image. These two images are then stereoscopically displayed to form a three-dimensional image of an object. Both shadow and reflective images can be used to form different perspectives of the object.

C. Image Creation

As set forth above in step 1840 (FIG. 18), acoustic signals transmitted from swallowable sensor device 104 can be used to create a three-dimensional image of an interior portion of patient 102. The three-dimensional image can be created based on the calculation of voxels. Example equations for calculating a voxel are described below.

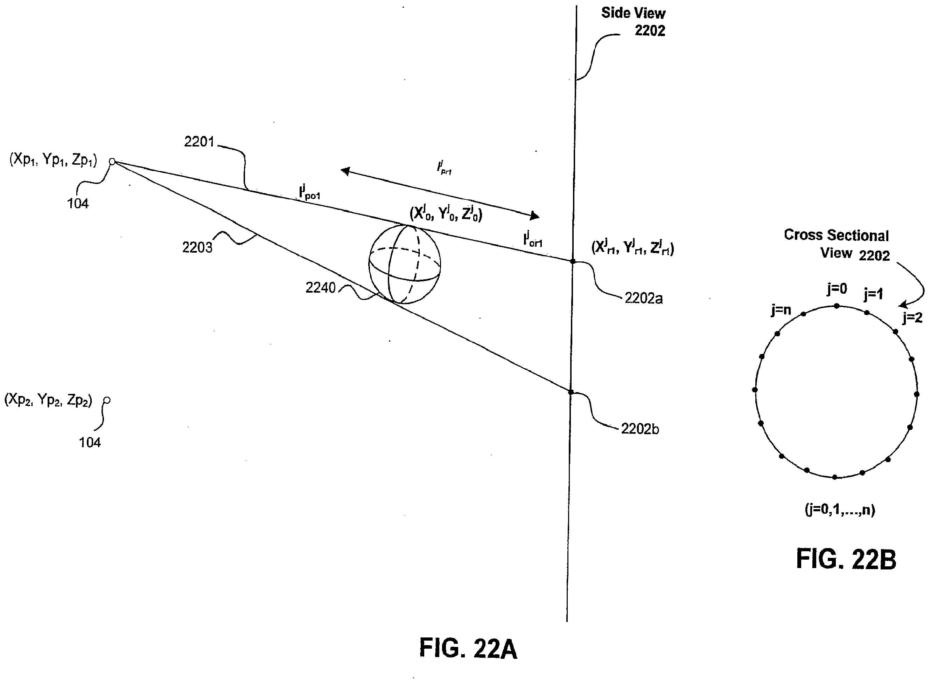

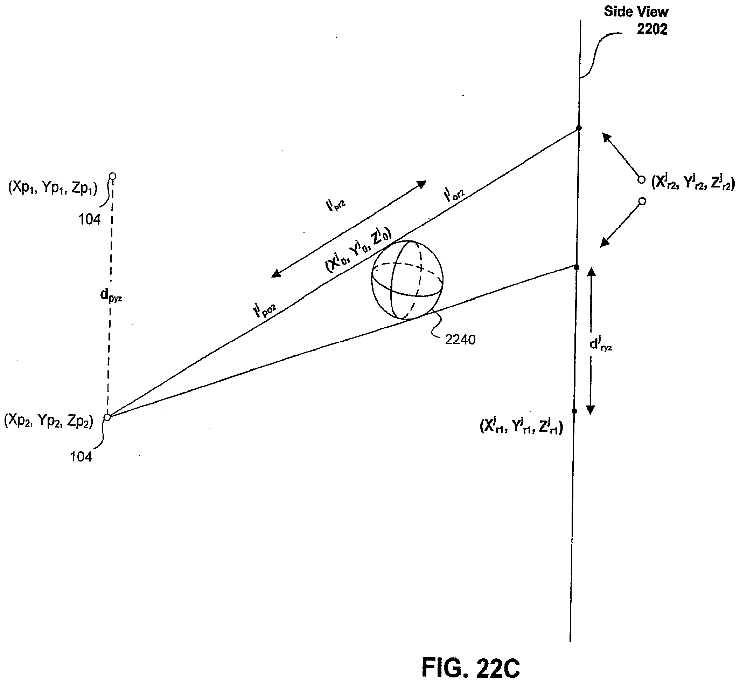

FIGS. 22A and 22C illustrate a plurality of sensing elements 2202, swallowable sensor device 104, and an object 2240 included in an interior portion of patient 102. In FIGS. 22A and 22C, swallowable sensor device 104 is illustrated when it is located at a first position $(x_{p1}, y_{p1}, z_{p1})$ and at a second position $(x_{p2}, y_{p2}, z_{p2})$. The plurality of sensing elements 2202 may be included on one or more sensor link modules 1202 that are adhesively coupled to patient 102 or may be included in a wearable fabric that patient 102 wears. Acoustic signals transmitted by swallowable sensor device 104 are received by sensing elements 2202 to compute coordinates $(x^j_o, y^j_o, z^j_o)$ of object 2240, as described in more detail below.

Referring to FIG. 22A, swallowable sensor device 104 can transmit a first acoustic signal at first location $(x_{p1}, y_{p1}, z_{p1})$. The first acoustic signal will propagate along multiple paths. A plurality of paths, from swallowable sensor device 104 to sensing elements 2202, are tangent to object 2240, such as a first path 2201 and a second path 2203.

The paths that are tangent to object 2240 can be distinguished from the other paths based on a difference in the amplitude of the first acoustic signal received by the plurality of sensing elements 2202. For example, sensing elements that are slightly below first sensing element 2202a will receive a signal having a slightly smaller amplitude compared to sensing elements that are slightly above first sensing element 2202a. The difference in amplitude is due to the partial reflection of the first acoustic signal as it impinges upon object 2240, as described above. Similarly, sensing elements that are slightly above second sensing element 2202b will receive a signal having a slightly smaller amplitude compared to sensing elements that are slightly below second sensing element 2202b.

In addition to the first and second sensing elements 2202a,b, a plurality of other sensing elements will receive the first acoustic signal along paths that are tangent to object 2240, as illustrated, for example, in FIG. 22B. The sensing elements that receive the first acoustic signal along these paths are labeled by the index $j$, wherein $j$ is an integer number that ranges from 0 to the total number of sensing elements that receive the first acoustic signal along a path tangent to object 2240.

The coordinates of these sensing elements - *i.e.*, those sensing elements which receive the first acoustic signal along paths that are tangent to object 2240 - are labeled $(x^j_{r1}, y^j_{r1}, z^j_{r1})$. The total distance from the first location of swallowable sensor device 104 to these sensing elements is labeled $l^j_{pr1}$. The total distance $l^j_{pr1}$ can be calculated, for example, by using one of the techniques described above in Section III above. The distance from swallowable sensor device 104 to the coordinates $(x^j_o, y^j_o, z^j_o)$ of object 2240 is labeled $l^j_{po1}$.

Referring to FIG. 22C, swallowable sensor device 104 can transmit a second acoustic signal at second location $(x_{p2}, y_{p2}, z_{p2})$. Similar to FIG. 22A, a plurality of sensing elements, labeled $(x^j_{r2}, y^j_{r2}, z^j_{r2})$, will receive the second acoustic signal after it traverses a path that is tangent to object 2240. The total distance of these paths is labeled $l^j_{pr2}$, and the distance from the second location of swallowable sensor device 104 to the coordinates $(x^j_o, y^jo, z^j_o)$ of object 2240 is labeled $l^j_{po2}$.

Between the first and second locations, swallowable sensor device 104 may have moved in a direction that is not parallel to sensing elements 2202. The distance that swallowable sensor device 104 moved in a direction parallel to sensing elements 2202 is labeled $d_{py1z1}$. The corresponding distance between sensing elements $(x^j_{r1}, y^j_{r1}, z^j_{r1})$ and $(x^j_{r2}, y^j_{r2}, z^j_{r2})$ is labeled $d^j_{ry1z1}$.

The distance $d_{pyz}$ is related to the distance $d^j_{ryz}$ by the following equation:

$$l^j_{po1} = \frac{l^j_{pr1}d_{py1z1}}{d^j_{ry1z1} + d_{py1z1}}$$ (Eq. 28)

wherein $l^j_{po1}$, $l^j_{pr1}$, $d_{py1z1}$, and $d^j_{ry1z1}$ represent the variables described above. Thus, Eq. 28 can be used to calculate the distance, $l^j_{po1}$, from swallowable sensor device 104 to object 2240 when swallowable sensor device 104 is at a first position.

Eq. 28 can be generalized to the following equation:

$$l^j_{poi} = \frac{l^j_{pri}d_{pyizi}}{d^j_{ryizi} + d_{pyizi}} ,$$ (Eq. 29)

wherein $i$ is a natural number that labels the positions of swallowable sensor device 104. Thus, Eq. 29 can be used to calculate the distance, $l^j_{poi}$, from swallowable sensor device 104 to object 2240 when swallowable sensor device 104 is at the *i*-th position.

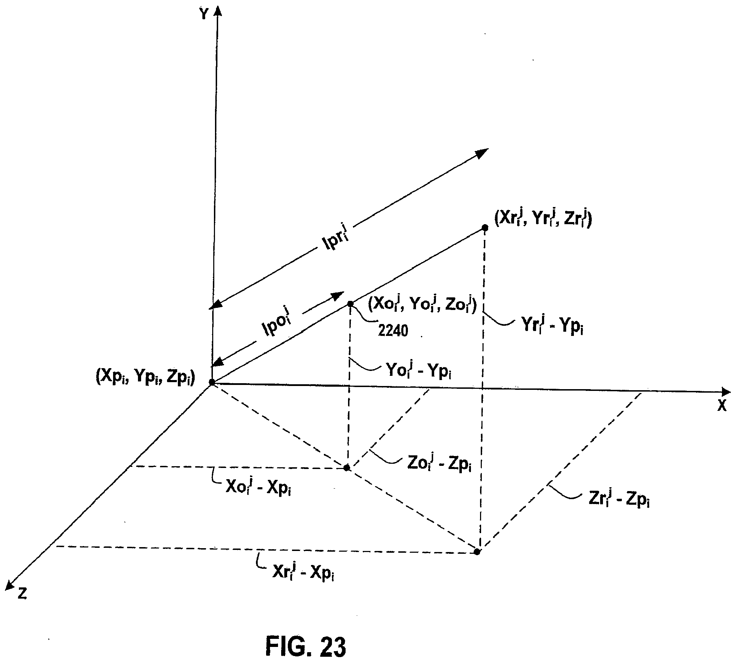

Based on the concept of similar triangles, the distance $l^j_{poi}$ can then be used to calculate the coordinates $(x^j_{oi}, y^j_{oi}, z^j_{oi})$ of object 2240, wherein these coordinates define the shape of object 2240. Example geometry for visualizing such similar triangles is depicted in FIG. 23. In FIG. 23, the coordinates $(x_{pi}, y_{pi}, z_{pi})$ represent the location of swallowable sensor device 104 when at the i-th position, the coordinates $(x^j_{oi}, y^j_{oi}, z^j_{oi})$ represent the point on the surface of object 2240 which is tangent to an acoustic signal that is transmitted from swallowable sensor device 104 when at the i-th position and that impinges on a j-th sensing element, and coordinates $(x^j_{ri}, y^j_{ri}, z^j_{ri})$ represent the position of the j-th sensing element.

To calculate $x^j_{oi}$, for example, the following similarity relationship is helpful:

$$\frac{x^j_{oi} - x_{pi}}{x^j_{ri} - x_{pi}} = \frac{l^j_{poi}}{l^j_{pri}} .$$ (Eq. 30)

Eq. 30 can be rearranged to yield

$$x^j_{oi} = x_{pi} + \left(x^j_{ri} - x_{pi}\right)\frac{l^j_{poi}}{l^j_{pri}}$$ (Eq. 31a)

Thus, Eq. 31a gives an x-coordinate of object 2240 (namely, $x^j_{oi}$) in terms of (1) the x-coordinate of swallowable sensor device 104 (namely, $x_{pi}$), (2) the x-coordinate of the j-th sensing element that receives an acoustic signal transmitted by swallowable sensor device 104 (namely, $x^j_{ri}$), (3) the distance from swallowable sensor device 104 when at position i to the j-th sensing element (namely, $l^j_{pri}$), and (4) the distance from swallowable sensor device 104 when at position i to object 2240 (namely, $l^j_{poi}$).

Analogous equations give a y- and z-coordinate of object 2240 :

$$y^j_{oi} = y_{pi} + \left(y^j_{ri} - y_{pi}\right)\frac{l^j_{poi}}{l^j_{pri}} \qquad (\text{Eq. 31b})$$

$$z^j_{oi} = z_{pi} + \left(z^j_{ri} - z_{pi}\right)\frac{l^j_{poi}}{l^j_{pri}} \qquad (\text{Eq. 31c})$$

The coordinates $\left(x^j_{0i}, y^j_{0i}, z^j_{0i}\right)$ of Eqs. 31a-c represent the three-dimensional volume elements of object 2240.

Thus, these coordinates can be used to form a three-dimensional image of object 2240.

In summary, acoustic signals transmitted from swallowable sensor device 104 can be used to calculate three-dimensional pixels, or voxels, of an interior portion of patient 102. First, the location of swallowable sensor device 104 can be determined using a locating technique, such as any of the locating techniques described above in Section III. Next, the location of object 2240 can be determined using Eq. 29. Then, the coordinates the surface of object 2240 can be calculated using Eq. 31a, 31b, and 31c. To calculate coordinates for the entire surface of object 2240, swallowable sensor device can transmit acoustic signals from multiple vantage points around object 2240. Based on these coordinates, a three-dimensional image of object 2240 can be formed.

The above-described calculations can be performed by a computation module embodied in control logic as would be apparent to a person skilled in the relevant art(s). For example, the calculations can be performed by a computation module included in external computing device 108, sensor link modules 602 or 1202, or swallowable sensor device 104.

V. Conclusion

Set forth above are example systems, methods, and apparatuses for locating a swallowable sensor device and imaging an internal portion of a patient using the swallowable sensor device. While various embodiments have been described above, it should be understood that they have been presented by way of example only, and not limitation. It will be apparent to persons skilled in the relevant art that various changes in form and detail can be made therein without departing from the spirit and scope of the description. For example, the swallowable sensor devices described herein may be swallowed by an animal to diagnose or aid in the diagnosis of one or more conditions of the animal. Such diagnosis may involve, for example, an immediate detection of a condition or attribute, or a historical and/or statistical analysis of multiple detections of conditions or attributes over a time period. Example embodiments described above relate to a human subject, for illustrative purposes. Embodiments are applicable to further types of animals other than humans, including livestock (cattle, sheep, pigs, chickens, turkeys, ostriches, etc.), pets (e.g., dogs, cats, horses, etc.), and other animals of interest such as race horses or other performance/sport animals. Such applicability to these types of animals, and other types, will be apparent to persons skilled in the relevant art(s) from the teachings herein, and is within the scope and spirit of embodiments. Furthermore, example embodiments described above relate to passing a swallowable sensor device through a gastrointestinal tract, for illustrative purposes. However, embodiments are applicable to further bodily systems other than the gastrointestinal tract, including the circulatory system, the urinary tract, and other bodily systems and additionally other means of entry or implant into a body cavity of an animal or human. Such applicability to other types of bodily systems will be apparent to persons skilled in the relevant art(s) from the teachings herein, and is within the scope and spirit of embodiments.

Furthermore, it should be understood that spatial descriptions (e.g., "above," "below," "up," "left," "right," "down," "top," "bottom," "vertical," "horizontal," etc.) used herein are for purposes of illustration only, and that practical implementations of the structures described herein can be spatially arranged in any orientation or manner.

The present invention is set out in the claims that follow. The embodiments, aspects or examples according to the present description that do not fall within the scope of said claims are provided for illustrative purposes only and do not form part of the present invention.

**Claims**

1. A method for locating a swallowable sensor device (104) disposed within a patient (102), comprising:

    (a) transmitting an acoustic signal (106) from the swallowable sensor device (104);
    (b) receiving the acoustic signal (106) at a plurality of sensing elements (802), the plurality of sensing elements receiving the acoustic signal at respective times;
    (c) establishing a reference time as the time when a first sensing element (802) receives the acoustic signal (106), the first sensing element receiving the acoustic signal before at least a subset of the other sensing elements; and
    (d) determining a location of the swallowable sensor device (104) based on the reference time and the respective times.

2. The method of claim 1, wherein step (c) comprises: establishing the reference time as the time when the sensing element (802) closest to the swallowable sensor device (104) receives the acoustic signal (106).

3. The method of claim 1, further comprising: (e) positioning the plurality of sensing elements (802) on a front portion of the patient (102).

4. The method of claim 3, wherein step (e) comprises: positioning the plurality of sensing elements (802) as a phased array on the front portion of the patient (102).

5. The method of claim 4, wherein step (d) comprises: (d1) determining an angle of incidence of the acoustic signal (106) as received by each of the sensing elements (802) based on the respective times when the acoustic signal (106) is received by the plurality of sensing elements (802); and (d2) computing the location of the swallowable sensor device (104) based on the reference time and the angles of incidence.

6. A system, comprising:

    a swallowable sensor device (104) adapted to be ingested by a patient (102), wherein the swallowable sensor device (104) transmits an acoustic signal (106);
    a plurality of sensing elements (802) adapted to be positioned on the patient (102), wherein the plurality of sensing elements (802) receive the acoustic signal (106) at respective times; and
    a computation module (108) that computes a location of the swallowable sensor device based on the respective times and a reference time, wherein the reference time is the time when a first sensing element (802) receives the acoustic signal (106), the first sensing element (802) receiving the acoustic signal (106) before at least a subset of the other sensing elements (802).

7. The system of claim 6, wherein the reference time is the time when the sensing element (802) closest to the swallowable sensor device (104) receives the acoustic signal (106).

8. The system of claim 6, wherein the plurality of sensing elements (802) are positioned on a front portion of the patient (102).

**Patentansprüche**

1. Verfahren zum Lokalisieren einer verschluckbaren Sensorvorrichtung (104), die sich in einem Patienten (102) befindet, wobei das Verfahren umfasst:

    (a) Übertragen eines akustischen Signals (106) von der verschluckbaren Sensorvorrichtung (104);
    (b) Empfangen des akustischen Signals (106) an einer Vielzahl von Sensorelementen (802), wobei die Vielzahl von Sensorelementen das akustische Signal zu jeweiligen Zeitpunkten empfangen;
    (c) Erstellen eines Referenzzeitpunkts als den Zeitpunkt, zu dem ein erstes Sensorelement (802) das akustische Signal (106) empfängt, wobei das erste Sensorelement das akustische Signal vor wenigstens einer Teilmenge der anderen Sensorelemente empfängt; und
    (d) Ermitteln der Position der verschluckbaren Sensorvorrichtung (104) basierend auf dem Referenzzeitpunkt und den jeweiligen Zeitpunkten.

**2.** Verfahren nach Anspruch 1, wobei Schritt (c) umfasst: Erstellen des Referenzzeitpunkts als den Zeitpunkt, zu dem das Sensorelement (802), das sich am nächsten an der verschluckbaren Sensorvorrichtung (104) befindet, das akustische Signal (106) empfängt.

**3.** Verfahren nach Anspruch 1, das des Weiteren umfasst: (e) Positionieren der Vielzahl von Sensorelementen (802) an einem vorderen Teil des Patienten (102).

**4.** Verfahren nach Anspruch 3, wobei Schritt (e) umfasst: Positionieren der Vielzahl von Sensorelementen (802) als Phasenarray am vorderen Teil des Patienten (102).

**5.** Verfahren nach Anspruch 4, wobei Schritt (d) umfasst: (d1) Bestimmen eines Einfallswinkels des akustischen Signals (106), wie es von jedem der Sensorelemente (802) empfangen wird, basierend auf den jeweiligen Zeitpunkten, zu denen das akustische Signal (106) von der Vielzahl von Sensorelementen (802) empfangen wird; und (d2) Berechnen der Position der verschluckbaren Sensorvorrichtung (104) basierend auf dem Referenzzeitpunkt und den Einfallswinkeln.

**6.** System, das aufweist:

eine verschluckbare Sensorvorrichtung (104), die dazu ausgelegt ist, von einem Patienten (102) eingenommen zu werden, wobei die verschluckbare Sensorvorrichtung (104) ein akustisches Signal (106) überträgt; eine Vielzahl von Sensorelementen (802), die dazu ausgelegt sind, an dem Patienten (102) positioniert zu werden, wobei die Vielzahl von Sensorelementen (802) das akustische Signal (106) zu jeweiligen Zeitpunkten empfangen; und ein Rechenmodul (108), das die Position der verschluckbaren Sensorvorrichtung basierend auf den jeweiligen Zeitpunkten und einem Referenzzeitpunkt berechnet, wobei der Referenzzeitpunkt der Zeitpunkt ist, zu dem ein erstes Sensorelement (802) das akustische Signal (106) empfängt, wobei das erste Sensorelement (802) das akustische Signal (106) vor wenigstens einer Teilmenge der anderen Sensorelemente (802) empfängt.

**7.** System nach Anspruch 6, wobei der Referenzzeitpunkt der Zeitpunkt ist, zu dem das Sensorelement (802), das sich am nächsten an der verschluckbaren Sensorvorrichtung (104) befindet, das akustische Signal (106) empfängt.

**8.** System nach Anspruch 6, wobei die Vielzahl von Sensorelementen (802) an einem vorderen Teil des Patienten (102) positioniert werden.

**Revendications**

**1.** Procédé permettant de localiser un dispositif de capteur avalable (104) disposé à l'intérieur d'un patient (102), consistant à :

(a) transmettre un signal acoustique (106) provenant du dispositif de capteur avalable (104) ;
(b) recevoir le signal acoustique (106) au niveau d'une pluralité d'éléments capteurs (802), la pluralité d'éléments capteurs recevant le signal acoustique à des moments respectifs ;
(c) établir un moment de référence en tant que moment où un premier élément capteur (802) reçoit le signal acoustique (106), le premier élément capteur recevant le signal acoustique avant au moins un sous-ensemble des autres éléments capteurs ; et
(d) déterminer une localisation du dispositif de capteur avalable (104) sur la base du moment de référence et des moments respectifs.

**2.** Procédé selon la revendication 1, dans lequel l'étape (c) consiste à : établir le moment de référence en tant que moment où l'élément capteur (802) le plus proche du dispositif de capteur avalable (104) reçoit le signal acoustique (106).

**3.** Procédé selon la revendication 1, consistant en outre à : (e) positionner la pluralité d'éléments capteurs (802) sur une partie avant du patient (102).

**4.** Procédé selon la revendication 3, dans lequel l'étape (e) consiste à : positionner la pluralité d'éléments capteurs (802) en tant que réseau phasé sur la partie avant du patient (102).

**5.** Procédé selon la revendication 4, dans lequel l'étape (d) consiste à : (d1) déterminer un angle d'incidence du signal acoustique (106) comme reçu par chacun des éléments capteurs (802) sur la base des moments respectifs où le signal acoustique (106) est reçu par la pluralité d'éléments capteurs (802) ; et (d2) calculer la localisation du dispositif de capteur avalable (104) sur la base du moment de référence et des angles d'incidence.

**6.** Système, comprenant :

un dispositif de capteur avalable (104) adapté pour être ingéré par un patient (102), dans lequel le dispositif de capteur avalable (104) transmet un signal acoustique (106) ;

une pluralité d'éléments capteurs (802) adaptés pour être positionnés sur le patient (102), dans lequel la pluralité d'éléments capteurs (802) reçoit le signal acoustique (106) à des moments respectifs ; et

un module de calcul (108) qui calcule une localisation du dispositif de capteur avalable sur la base des moments respectifs et d'un moment de référence, dans lequel le moment de référence est le moment où un premier élément capteur (802) reçoit le signal acoustique (106), le premier élément capteur (802) recevant le signal acoustique (106) avant au moins un sous-ensemble des autres éléments capteurs (802).

**7.** Système selon la revendication 6, dans lequel le moment de référence est le moment où l'élément capteur (802) le plus proche du dispositif de capteur avalable (104) reçoit le signal acoustique (106).

**8.** Système selon la revendication 6, dans lequel la pluralité d'éléments capteurs (802) est positionnée sur une partie avant du patient (102).

**FIG. 1**

FIG. 2

communications module

acoustic
communications module

204

302

radiating element

304

# FIG. 3

FIG. 4

**FIG. 5**

EP 2 063 780 B1

**FIG. 6**

EP 2 063 780 B1

602

702

control logic

706

708

106

704

sensor
communication
module

storage

remote
communication
module

604

110

710

power source

FIG. 7

**FIG. 8**

802

902

Transducer

FIG. 9

902

1004

detector ◄ - - v

106

1004

FIG. 10

Computer System **1100**

**FIG. 11**

**FIG. 12**

1300

Transmit an acoustic signal
from a swallowable sensor
device
1310

Receive the acoustic signal
at a plurality of sensing
elements located outside a
patient's body
1320

1340
Determine a location of the
swallowable sensor device
based on an angle of
incidence of the received
acoustic signal

1350
Determine a location of the
swallowable sensor device
based on a reference time
established

FIG. 13

**FIG. 14**

EP 2 063 780 B1

**FIG. 15**

EP 2 063 780 B1

**FIG. 16**

**FIG. 17**

1800

Transmit a first signal and a second signal — 1810

Receive the first and second signals at a plurality of sensing elements — 1820

Display first and second two-dimensional images to form a stereoscopic image of an interior portion of a patient

1830

1840

Calculate a three-dimensional image of an interior portion of a patient based on the first and second received signals

FIG. 18

**FIG. 19A**

**FIG. 19B**

swallowable sensor
device 104

body

$(Z_b, C_b)$

2001

2002

outside body

$(t_1, x_1, y_1, z_1)$

$(t_2, x_2, y_2, z_2)$

2003

2002b

$(t_3, x_3, y_3, z_3)$

$s_t$

object

$(Z_{ob}, C_{ob})$

$s_o$

$s_i$

$s_r$

1940

2005

$(t_4, x_4, y_4, z_4)$

$(t_5, x_5, y_5, z_5)$

2002a

FIG. 20A

EP 2 063 780 B1

FIG. 20B

**FIG. 21A**

**FIG. 21B**

FIG. 22A

Cross Sectional View 2202

(j=0,1,...,n)

FIG. 22B

FIG. 22C

FIG. 23

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20040068204 A
- US 7160258 B, Imran